# EUROPEAN PATENT SPECIFICATION

(11) **EP 2 548 030 B1**
(45) Date of publication and mention of the grant of the patent: **27.05.2015**
(21) Application number: 11757053.1
(22) Date of filing: 18.03.2011
(51) Int. Cl.: C12N 15/10, G01N 33/68

(54) **ENGINEERING CORRECTLY FOLDED ANTIBODIES USING INNER MEMBRANE DISPLAY OF TWIN-ARGININE TRANSLOCATION INTERMEDIATES**
ENGINEERING KORREKT GEFALTETER ANTIKÖRPER MIT MEMBRANINTERNER ANZEIGE VON TWIN-ARGININ-TRANSLOKATIONS-ZWISCHENPRODUKTEN
PRÉPARATION D'ANTICORPS CORRECTEMENT PLIÉS UTILISANT LA PRÉSENTATION SUR LA MEMBRANE INTÉRIEURE D'INTERMÉDIAIRES DE TRANSLOCATION TWIN-ARGININE

(30) Priority: 18.03.2010 US 315088 P
(43) Date of publication of application: 23.01.2013
(73) Proprietor: Cornell University, Ithaca, NY 14850 (US)
(72) Inventor: DELISA, Matthew, Ithaca, NY 14850 (US); LIM, Hyung-Kwon, Seongnam Gyeonggi 463-772 (KR); KARLSSON, Amy, Ithaca, NY 14850 (US)
(74) Representative: Glawe, Delfs, Moll
(86) International application number: PCT/US2011/028977
(87) International publication number: WO 2011/116276

(56) References cited:
- WO-A1-2004/050871
- WO-A1-2010/057097
- WO-A2-03/040335
- WO-A2-2007/016591
- WO-A2-2007/024877
- WO-A2-2008/110914
- WO-A2-2008/156551
- US-A1- 2005 249 748
- US-A1- 2009 220 952
- US-B2- 7 419 783
- FISHER A C ET AL: "Efficient Isolation of Soluble Intracellular Single-chain Antibodies using the Twin-arginine Translocation Machinery", JOURNAL OF MOLECULAR BIOLOGY, ACADEMIC PRESS, UNITED KINGDOM, vol. 385, no. 1, 9 January 2009 (2009-01-09), pages 299-311, XP025846140, ISSN: 0022-2836, DOI: 10.1016/J.JMB.2008.10.051 [retrieved on 2008-11-01]
- FISHER ADAM C ET AL: "Genetic selection for protein solubility enabled by the folding quality control feature of the twin-arginine translocation pathway", PROTEIN SCIENCE, WILEY, US, vol. 15, no. 3, 1 March 2006 (2006-03-01), pages 449-458, XP002524305, ISSN: 0961-8368, DOI: 10.1110/PS.051902606
- STEFAN LUTZ ET AL: "A universal, vector-based system for nucleic acid reading-frame selection", PROTEIN ENGINEERING, OXFORD UNIVERSITY PRESS, SURREY, GB, vol. 15, no. 12, 1 January 2002 (2002-01-01), pages 1025-1030, XP008125589, ISSN: 0269-2139
- ADAM C. FISHER ET AL: "Exploration of twin-arginine translocation for expression and purification of correctly folded proteins in Escherichia coli", MICROBIAL BIOTECHNOLOGY, vol. 1, no. 5, 1 September 2008 (2008-09-01), pages 403-415, XP55025968, ISSN: 1751-7907, DOI: 10.1111/j.1751-7915.2008.00041.x
- EVA-MARIA STRAUCH ET AL: "A bacterial two-hybrid system based on the twin-arginine transporter pathway of E. coli", PROTEIN SCIENCE, vol. 16, no. 5, 1 May 2007 (2007-05-01), pages 1001-1008, XP55025965, ISSN: 0961-8368, DOI: 10.1110/ps.062687207
- KOSTAS HATZIXANTHIS ET AL: "A subset of bacterial inner membrane proteins integrated by the twin-arginine translocase", MOLECULAR MICROBIOLOGY, vol. 49, no. 5, 1 September 2003 (2003-09-01), pages 1377-1390, XP55026069, ISSN: 0950-382X, DOI: 10.1046/j.1365-2958.2003.03642.x
- PHILIBERT PASCAL ET AL: "A focused antibody library for selecting scFvs expressed at high levels in the cytoplasm", BMC BIOTECHNOLOGY, BIOMED CENTRAL LTD. LONDON, GB, vol. 7, 22 November 2007 (2007-11-22), pages 1-17, XP002509301, ISSN: 1472-6750, DOI: 10.1186/1472-6750-7-81
- FISHER, ADAM C. ET AL.: 'Folding quality control in the export of proteins by the bacterial twin-arginine translocation pathway' PNAS vol. 100, no. 10, 13 May 2003, pages 6115 - 6120, XP002970386

## Description

### Field of the Invention

The present invention relates to systems, vectors and methods for isolation of enhanced ligand-binding proteins from combinatorial libraries displayed on the inner membrane of a host cell.

### Background of the Invention

The expression of heterologous proteins represents a cornerstone of the biotechnology enterprise. Unfortunately, many commercially important proteins misfold and aggregate when expressed in a heterologous host (See, e.g., Makrides, Microbiol Rev 60, 512-538 (1996); Baneyx and Mujacic, Nat Biotechnol 22, 1399-1408 (2004); Georgiou and Valax, Curr Opin Biotechnol 7, 190-197 (1996)). Existing biochemical means for assessing the tendency of proteins to misfold and aggregate are tedious. As a result, screening for constructs and/or conditions that favor solubility is inefficient and genetic selection of folded structures has not been forthcoming.

Development of a robust assay for *in vivo* protein folding and solubility has been challenging for researchers because of limitations on detecting and reporting the solubility of a protein. Existing systems for monitoring protein misfolding *in vivo* have capitalized on the observation that a misfolded target protein will often co-translationally induce improper folding of a C-terminally fused reporter protein (See, e.g., Maxwell et al., Protein Sci 8, 1908-1911 (1999); Waldo et al., Nat Biotechnol 17, 691-695 (1999)) or protein fragment (See, e.g., Cabantous et al., Nat Biotechnol 23, 102-107 (2005); Wigley et al., Nat Biotechnol 19, 131-136 (2001)) or will induce a specific gene response (See, e.g., Lesley et al., Protein Eng 15, 153-160 (2002)). This fusion approach is often problematic as certain reporter proteins can remain active even when the target protein to which they are fused aggregates or forms inclusion bodies (See, e.g., Tsumoto et al., Biochem Biophys Res Commun 312, 1383-1386 (2003)) while the gene expression response is limited by its indirect connection to the folding process.

Additionally, existing assays for protein expression in soluble form are tedious, usually requiring lysis and fractionation of cells followed by protein analysis by SDS-polyacrylamide gel electrophoresis. Using these traditional approaches, screening for protein constructs and/or physiological conditions yielding improved solubility is inefficient, and genetic selection nearly impossible.

A number of systems have been developed for improving the binding affinity of proteins such as antibodies. These systems generally use iterative rounds of mutagenesis and panning phages that display the antibody. However, these systems have drawbacks because the selected antibody may have a high binding affinity but may be impossible or difficult to express in a host cell due to misfolding of the antibody.

What is needed in the art are methods and systems for simultaneously selecting binding proteins that have a high binding affinity and which are also efficiently folded and expressed.

### Summary of the Invention

The present invention is defined in the claims and provides systems, vectors and methods for isolation of enhanced ligand-binding proteins from combinatorial libraries displayed on the inner membrane of a host cell.

For example, in some embodiments, the present invention provides a method of screening mutants of a target protein for a desired property comprising: displaying a library of heterologous fusion proteins on the inner membrane of host cells (e.g., where each host cell expresses a single species of fusion protein), wherein the heterologous fusion proteins comprises segments encoding a Tat signal operably linked to a target protein, wherein the Tat signal sequence is on the N-terminal of the fusion protein; forming spheroplasts from the host cells; contacting the spheroplasts with a target protein binding partner; and selecting spheroplasts that bind the target protein binding partner. In some embodiments, the library of heterologous fusion proteins comprises mutagenized target proteins. The present invention is not limited to particular target proteins. For example, in some embodiments, target proteins are antigen binding proteins (e.g., a scFV or an intrabody), receptor proteins or receptor ligand proteins. The present invention is not limited to particular target protein binding partners. For example, in some embodiments, the target protein binding partner is a molecule comprising an epitope, a receptor protein, or a receptor ligand. In some embodiments, the target protein binding partner is displayed on a solid support (e.g., beads or planar solid supports). In some embodiments, mutagenized target proteins are encoded by nucleic acids mutagenized by an amplification based mutagenesis procedure. In some embodiments, contacting the spheroplasts with a target protein binding partner includes contacting with a competitive binding partner. In some embodiments, the fusion protein comprises a protein tag at the C-terminus of the fusion protein. In some embodiments, the method comprises contacting the spheroplasts with a reagent specific for the protein tag, wherein the selecting further comprises selecting spheroplasts that bind both the binding partner and the reagent specific for the protein tag. In some embodiments, the spheroplasts that express target proteins with desired properties are isolated by flow cytometry, fluorescent activated cell sorting, or similar methods.

In some embodiments, the method further comprises the step of isolating DNA encoding the mutagenized target protein from the spheroplasts that bind the target protein binding partner (e.g., by subjecting DNA encoding the mutagenized target protein to a second round of mutagenesis to provide a second mutagenized target protein nucleic acid library, expressing the second mutagenized target protein nucleic acid library in a host cell, forming spheroplasts from the host cells; contacting the spheroplasts with target protein binding partner; and selecting spheroplasts that bind the target protein binding partner). In some embodiments, DNA encoding the mutagenized target protein is isolated from the spheroplasts that bind the target protein binding partner. In some embodiments, the mutagenized protein exhibits a property selected from, for example, enhanced solubility, intracellular folding efficiency, binding affinity for the target protein binding partner, and combinations thereof.

In further embodiments, the present invention provides a method of screening mutants of a target protein for a desired property comprising: a) expressing in host cells a library of target nucleic acid molecules encoding fusion proteins comprising a Tat signal sequence operably linked to a mutated target protein so that the fusion proteins are displayed on the inner membrane of the host cells (e.g., wherein each host cell expresses a single species of mutated target protein); b) forming spheroplasts from the host cells; c) contacting the spheroplasts with a target protein binding partner; d) selecting spheroplasts that bind the target protein binding partner; and e) isolating a target nucleic acid molecule encoding the mutated target protein from the spheroplasts that bind the target protein binding partner, wherein the mutagenized protein exhibits a property selected from the group consisting of enhanced solubility, intracellular folding efficiency, binding affinity for the target protein binding partner, and combinations thereof. In some embodiments, the method further comprises the step of f) mutagenizing the nucleic acid molecule isolated in step e and operably linking the nucleic acid molecules to a Tat signal sequence to provide a second library of mutagenized target nucleic acid molecules, and g) repeating steps b-e. In other embodiments, the method further comprises the step of f) mutagenizing the nucleic acid molecule isolated in step g and operably linking the nucleic acid molecules to a Tat signal sequence to provide a third library of mutagenized target nucleic acid molecules, and g) repeating steps b-e. In some embodiments, the mutagenized target proteins are for example, antigen binding proteins, receptor proteins (e.g., an scFV or an intrabody) or receptor ligand proteins. The present invention is not limited to particular target protein binding partners. For example, in some embodiments, the target protein binding partner is a molecule comprising an epitope, a receptor protein, or a receptor ligand. In some embodiments, the target protein binding partner is displayed on a solid support (e.g., beads or planar solid supports).

A nucleic acid isolated by any of the aforementioned methods and/or a mutant target protein encoded by the nucleic acid may be provided.

In additional embodiments, the present invention provides a method of screening mutants of a target protein for one or more desired properties comprising: a) expressing in host cells a library of target nucleic acid molecules encoding fusion proteins comprising a Tat signal sequence and protein tag operably linked to a mutated target protein so that the fusion proteins are displayed on the inner membrane of the host cells (e.g., wherein each host cell expresses a single species of mutated target protein), b) forming spheroplasts from the host cells; c) contacting the spheroplasts with a target protein binding partner and reagent specific for the protein tag; d) selecting spheroplasts that bind the target protein binding partner and the regaent specific for the protein tag; and e) isolating a target nucleic acid molecule encoding the mutated target protein from the spheroplasts that bind the target protein binding partner, wherein the mutagenized protein exhibits a property selected from the group consisting of enhanced solubility, intracellular folding efficiency, binding affinity for the target protein binding partner, and combinations thereof.

The present invention also relates to a system for screening mutants of a target protein for a desired property comprising: spheroplasts comprising a library of target nucleic acid molecules encoding fusion proteins comprising a Tat signal sequence operably linked to a mutated target protein so that the fusion proteins are displayed on the inner membrane of the spheroplasts (e.g., wherein each spheroblast expresses a single species of mutated target protein); and a solid support comprising a binding partner for the mutated target protein.

In still further embodiments, the present invention provides a library of spheroplasts comprising a library of target nucleic acid molecules encoding heterologous fusion proteins comprising a Tat signal sequence operably linked to a mutated target protein so that the fusion proteins are displayed on the inner membrane of the spheroplasts (e.g., wherein each host cell expresses a single species of mutated target protein).

### Description of the Figures

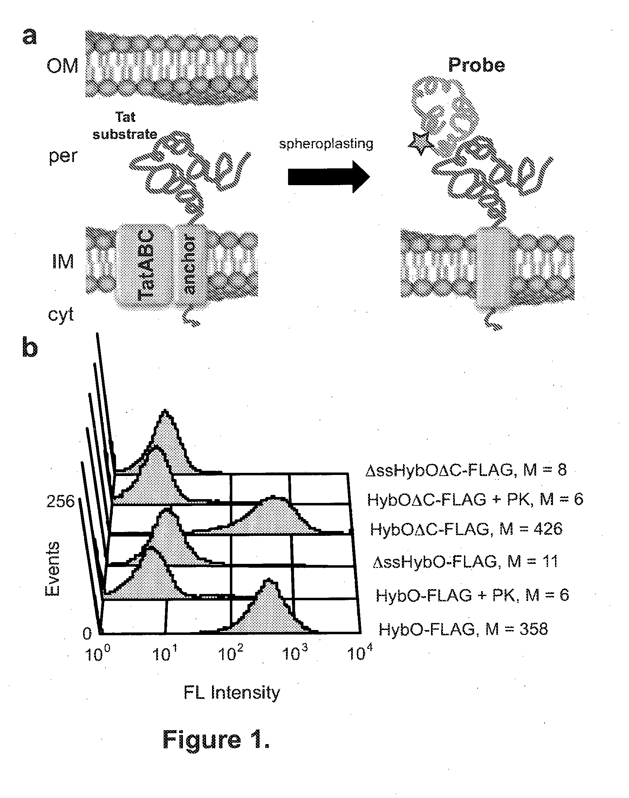

Figure 1. IM-anchored display of Tat substrates. (a) Correctly folded Tat substrates are transported from the cytoplasm (cyt) to the periplasm (per) of E. coli, but remain N-terminally anchored to the inner membrane (IM). After removing the outer membrane (OM) and periplasm, the protein can be detected by immunolabeling and/or probed for interactions with other proteins. (b) FC analysis of spheroplasts expressing HybO constructs with C-terminal FLAG epitope tags. Constructs lacking a native Tat signal peptide (ΔssHybO) and/or a C-tail membrane anchor (HybOΔC) were included as controls. Specified samples were treated with proteinase K (PK). FLAG tags were detected using a FITC-conjugated anti-FLAG antibody. Median fluorescence values (M) are shown for each construct.

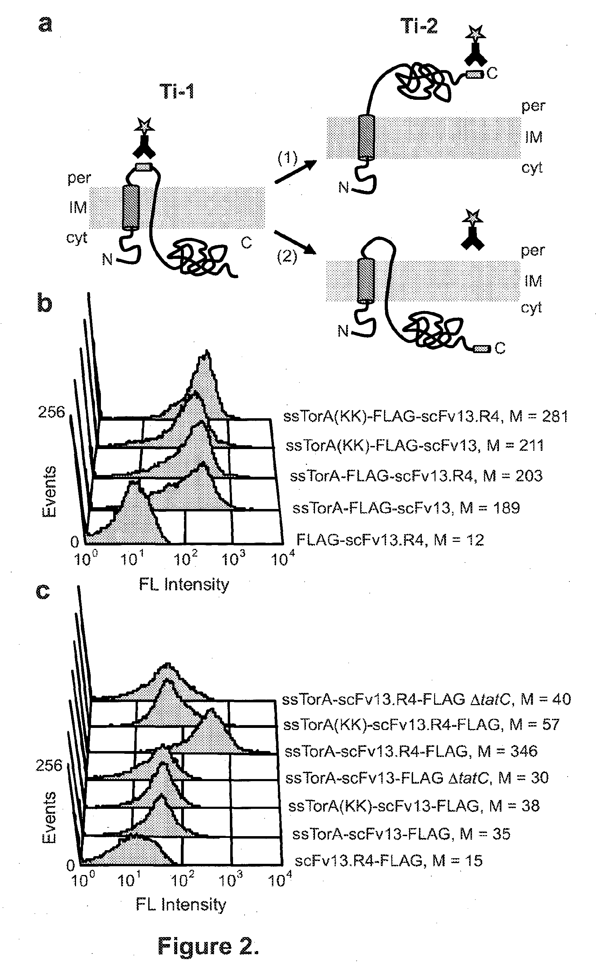

Figure 2. Separate detection of Ti-1 and Ti-2. (a) Schematic of Tat translocation intermediates. Formation of Ti-1 can be detected when an epitope tag is inserted between the signal peptide and the N-terminus of the protein. If the protein is incorrectly folded (2), it cannot form Ti-2 and a C-terminal epitope tag is not accessible for immunolabeling. If the protein is properly folded (1), it is transported to the periplasm to form Ti-2, and a C-terminal epitope tag can be detected on the periplasmic face of the inner membrane. (b) FC analysis to detect Ti-1 for a poorly folded scFv(scFv13) and a well folded scFv (scFv13.R4). FLAG tags were inserted between the N-terminal Tat signal peptide and the scFvs. (c) FC analysis to detect Ti-2 for scFv13 and scFv13.R4. FLAG tags were placed at the C-terminus of scFvs. ssTorA(KK) indicates mutation of the Arg-Arg motif to Lys-Lys in the Tat signal peptide; ΔtatC indicates cells that lacked the TatC protein. FLAG tags were detected with a FITC-conjugated anti-FLAG antibody, and ssTorA-scFv13 lacking an epitope tag was included as a control. Median fluorescence values (M) are shown.

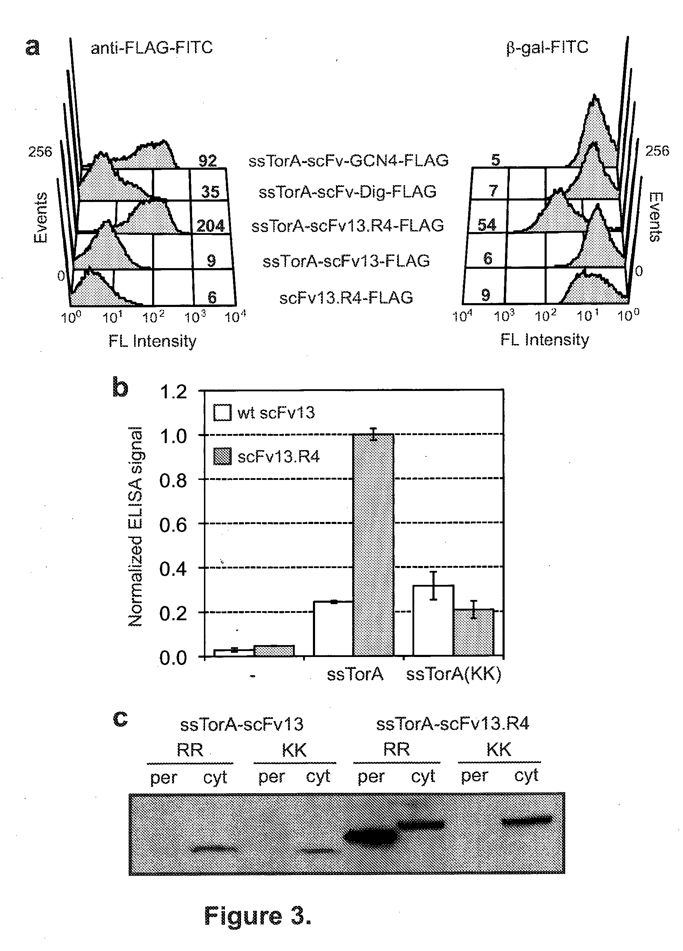

Figure 3. Expression and activity of IM-anchored scFvs. (a) FC analysis to detect Ti-2 formation and antigen binding activity for the scFvs. The antigen used was FITC-labeled β-gal. Poorly folded scFvs (scFv13 and scFv-Dig) and scFvs specific for irrelevant antigens (scFv-Dig and scFv-GCN4) were included. scFv13.R4-FLAG lacking a Tat signal peptide was used as a negative control. FLAG tags were detected with a FITC-conjugated anti-FLAG antibody. Median fluorescence values are shown directly in histograms. (b) Detection of ligand binding by ELISA for spheroplasts displaying scFv13-FLAG and scFv13.R4-FLAG. Binding activity was measured using β-gal-coated ELISA plates. Bound scFvs were detected with an anti-FLAG antibody. ELISA signals were normalized to the signal for scFv13.R4. Hyphen (-) indicates constructs lacking a signal peptide; KK indicates an Arg-Arg to Lys-Lys substitution in ssTorA. Data represents the average of three replicates, and error bars represent standard error of the mean. (c) Western blot analysis of periplasmic and cytoplasmic fractions from cells expressin scFv13 and scFv13.R4 fused to either ssTorA or ssTorA(KK). Blot was probed with an anti-FLAG antibody. An equivalent number of cells was loaded in each lane.

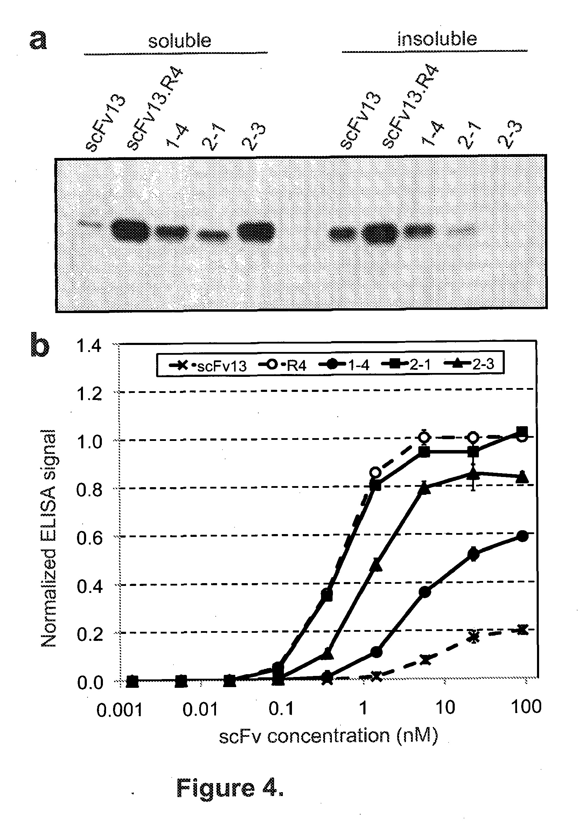

Figure 4. Expression and activity of scFv clones isolated using MAD-TRAP. (a) Western blot analysis of soluble and insoluble fractions from cells expressing scFvs in the cytoplasm. Clone 1-19 4, 2-1 and 2-3 were isolated using MAD-TRAP. scFv13 was the starting sequence for the first round library, and scFv13.R4 was isolated in a previous study after four rounds of directed evolution (Martineau et al., (1998) J Mol Biol 280(1):117-127). Samples were normalized by total protein concentration in the soluble fraction, and blot was probed with an anti-6x-His antibody. (b) ELISA data for binding of isolated clones to β-gal. scFvs were purified from cell lysate, and their binding to β-gal-coated ELISA plates was measured. Bound scFvs were detected with an anti-6x-His antibody. Data represents the average of six replicates and are normalized to the signal for scFv13.R4 at ∼20 nM. Error bars represent standard error of the mean.

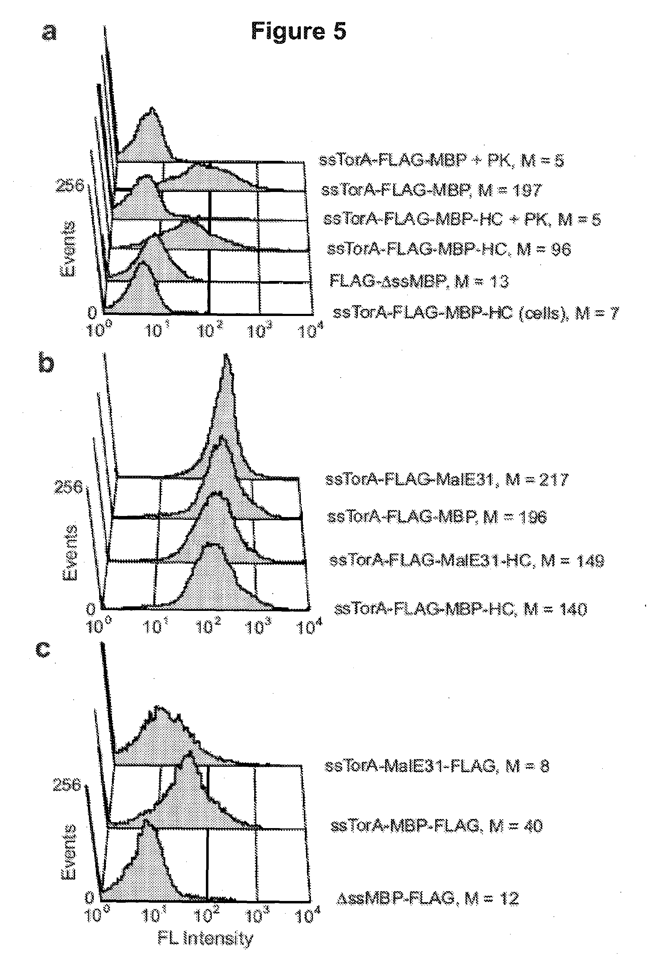

Figure 5. IM-anchored display of MBP. (a) FC analysis of MBP constructs with and without a C-terminal HybO C-tail (HC). FLAG tags were placed between the ssTorA signal peptide and MBP. Specified samples were treated with proteinase K (PK). Constructs lacking a signal peptide (ΔssMBP) and cells that were not spheroplasted were included as controls. (b) FC analysis of misfolding MBP variant. An aggregation-prone MBP variant (MalE31) and wt MBP were expressed with and without the C-terminal HC. FLAG tags were between the ssTorA signal peptide and MBP. (c) FC analysis of MBP constructs after repositioning of the FLAG tag to the C-terminus of wt MBP and MalE31. For (a)-(c), FLAG tags were detected with a FITC-conjugated anti-FLAG antibody, and median fluorescence values (M) are shown.

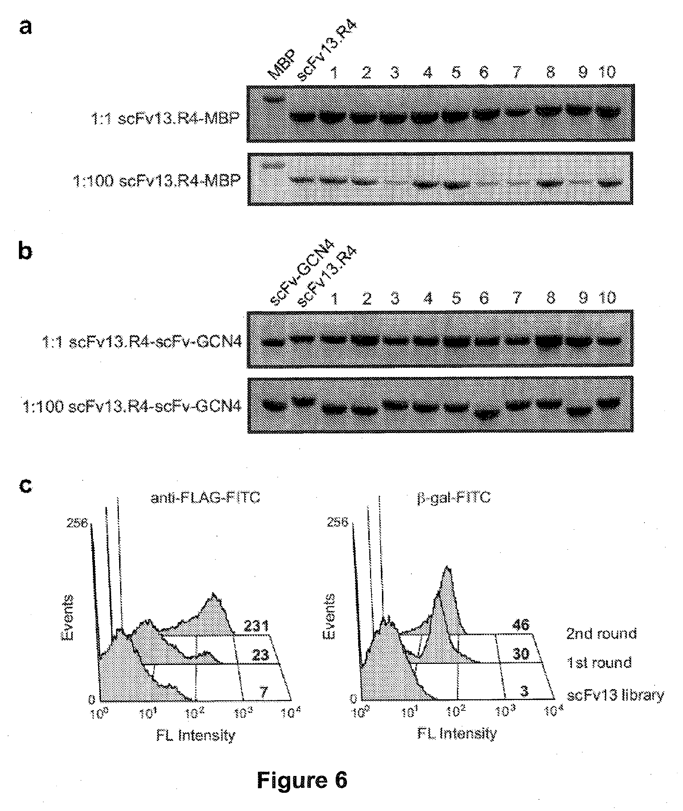

Figure 6. Library screening using MAD-TRAP. (a) PCR analysis of colonies isolated from mixtures of scFv13.R4 and MBP. Spheroplasts of cells expressing scFv13.R4 and MBP were mixed at ratios of 1:1 and 1:100 and panned against -gal beads. After amplification from bead-bound spheroplasts, PCR products were analyzed by gel electrophoresis to determine the identity of the isolated proteins. (b) PCR analysis of colonies isolated from libraries of scFv13.R4 and scFv-GCN4. Spheroplasts of cells expressing scFv13.R4 and scFv-GCN4 were mixed at ratios of 1:1 and 1:100 and panned against -gal beads. After amplification from bead-bound spheroplasts, PCR products were analyzed by gel electrophoresis to determine the identity of the isolated proteins. (c) FC analysis scFv13 random mutagenesis library panning. Spheroplasts expressing the initial scFv13 random mutagenesis library and spheroplasts expressing the libraries resulting from the first and second round of β-gal bead panning were interrogated for the presence of Ti-2 using an anti-FLAG FITC antibody and for antigen binding activity using β-gal-FITC. Median fluorescence values are shown directly in the histogram.

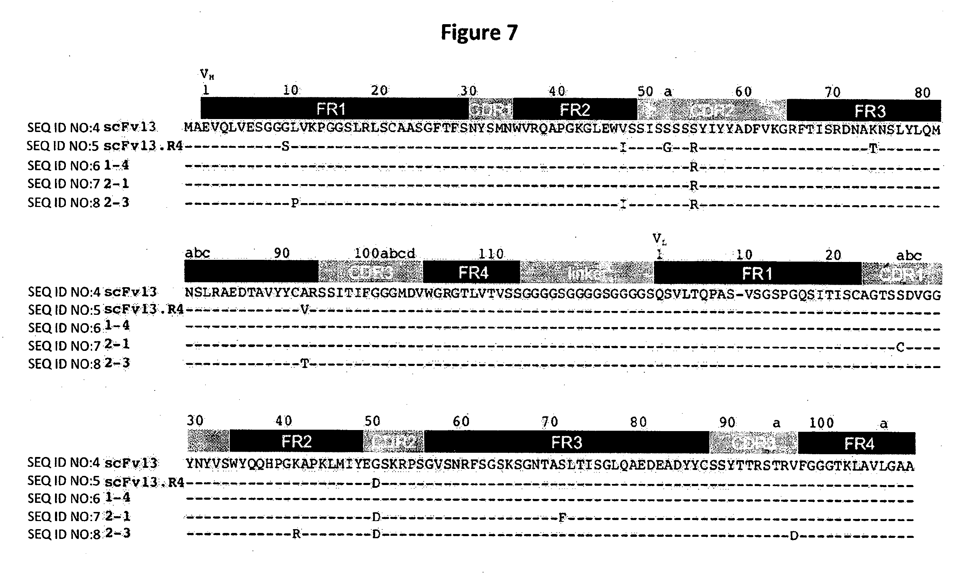

Figure 7. Amino acid sequences of anti- -gal scFv clones isolated using MADTRAP. Sequences for scFv13.R4, clone 1-4 from round 1, and clones 2-1 and 2-3 from round 2 are aligned with the sequence for scFv13. scFv13 was the parent scFv for the random mutagenesis library, and scFv13.R4 was isolated in a previous study after four rounds of directed evolution (Martineau et al., 1998, supra). Sequences are numbered using the Kabat numbering system (Abhinandan and Martin, 2008). The scFv sequences begin with the heavy chain (VH), which is linked to the light chain (VL) by a flexible amino acid linker.

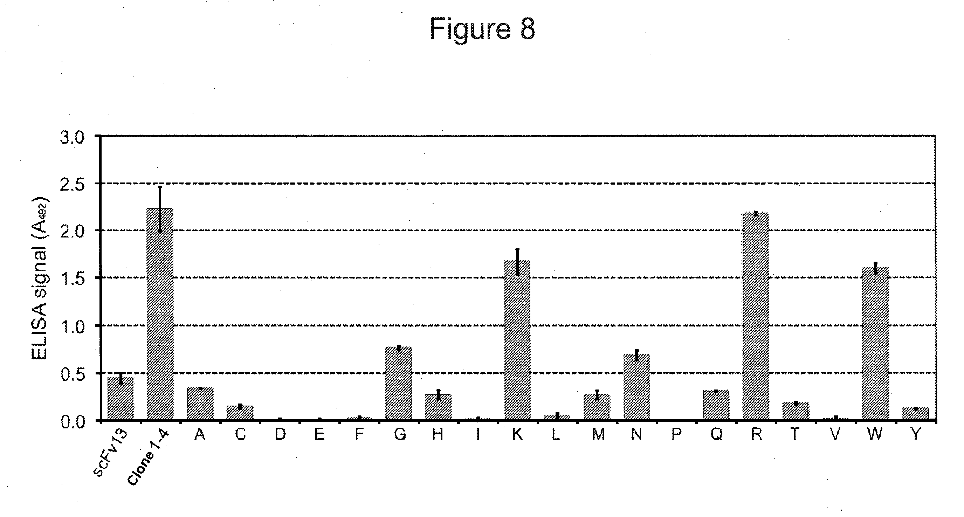

Figure 8. ELISA data for binding activity of saturation mutagenesis clones. Residue S55 in the VH domain of scFv13 was mutated to all 20 amino acids using a random NNK mutagenesis strategy. Cells expressing each of the scFv S55X (where X is the amino acid indicated) variants in the cytoplasm were lysed to obtain the soluble fraction. Samples were normalized by total protein concentration in the soluble fraction. scFvs bound to -gal were detected with an anti-6x-His antibody. Data are the average of three replicates, and error bars represent standard error of the mean.

### Definitions

To facilitate an understanding of the invention, a number of terms are defined below.

As used herein, the term "Tat signal sequence" refers to sequences that are recognized for export by the twin-arginine translocation (Tat) pathway. The Tat pathway serves the role of transporting folded proteins across energy-transducing membranes. Homologues of the genes that encode the transport apparatus occur in archaea, bacteria, chloroplasts, and plant mitochondria. In bacteria, the Tat pathway catalyses the export of proteins from the cytoplasm across the inner/cytoplasmic membrane. In chloroplasts, the Tat components are found in the thylakoid membrane and direct the import of proteins from the stroma. The Tat pathway acts separately from the general secretory (Sec) pathway, which transports proteins in an unfolded state.

It is generally accepted that the primary role of the Tat system is to translocate fully folded proteins across membranes. An example of proteins that need to be exported in their 3D conformation are redox proteins that have acquired complex multi-atom cofactors in the bacterial cytoplasm (or the chloroplast stroma or mitochondrial matrix). They include hydrogenases, formate dehydrogenases, nitrate reductases, trimethylamine N-oxide (TMAO) reductases and dimethyl sulphoxide (DMSO) reductases. The Tat system can also export whole heteroligomeric complexes in which some proteins have no Tat signal. This is the case of the DMSO reductase or formate dehydrogenase complexes. But there are also other cases where the physiological rationale for targeting a protein to the Tat signal is less obvious. Indeed, there are examples of homologous proteins that are in some cases targeted to the Tat pathway and in other cases to the Sec apparatus. Some examples are: copper nitrite reductases, flavin domains of flavocytochrome c and N-acetylmuramoyl-L-alanine amidases.

The Tat signal peptide consists of three motifs: the positively charged N-terminal motif, the hydrophobic region and the C-terminal region that generally ends with a consensus short motif (A-x-A) specifying cleavage by signal peptidase. Sequence analysis revealed that signal peptides capable of targeting the Tat protein contain the consensus sequence [ST]-R-R-x-F-L-K. The nearly invariant twin-arginine gave rise to the pathway's name. In addition the h-region of Tat signal peptides is typically less hydrophobic than that of Sec-specific signal peptides .

Proteins assembled with various cofactors or by means of cytosolic molecular chaperones are poor candidates for translocation across the bacterial inner membrane by the standard general secretory (Sec) pathway. This entry describes a family of predicted long, non-Sec signal sequences and signal-anchor sequences (uncleaved signal sequences). A large fraction of the members of this family may have bound redox-active cofactors.

Examples of Tat signal sequences include, but are not limited, TorA, CueO, DmsA, FdnG, FdoG, HyaA, NapA, Sufl, TorA, WcaM, YagT, YcbK, YcdB, YdhX, and YnfE Tat signal sequences.

As used herein, the term "target protein" when used in reference to a protein or nucleic acid refers to a protein or nucleic acid encoding a protein of interest for which solubility and/or folding is to be analyzed and/or altered of the present invention. The term "target protein" encompasses both wild-type proteins and those that are derived from wild type proteins (e.g., variants of wild-type proteins or polypeptides, or, chimeric genes constructed with portions of target protein coding regions), and further encompass fragments of a wild-type protein. Thus, in some embodiments, a "target protein" is a variant or mutant, such as a mutant produced via a directed evolution process. The present invention is not limited by the type of target protein analyzed.

As used herein, the term "fusion protein" refers to a polypeptide sequence, and nucleic acid molecules encoding the same, comprising segments from at least two heterologous polypeptides or proteins, for example, a Tat signal sequence operably linked to a heterologous target sequence. Multiple Tat signal seqeunces are known in the art and are contemplated to be useful in the present invention. The present invention contemplates that the fusion protein may be under the control of an inducible, a constitutively active, or other promoter.

As used herein, the term "gene transfer system" refers to any means of delivering a composition comprising a nucleic acid sequence to a cell or tissue. For example, gene transfer systems include, but are not limited to, vectors (e.g., retroviral, adenoviral, adeno-associated viral, and other nucleic acid-based delivery systems), microinjection of naked nucleic acid, polymer-based delivery systems (e.g., liposome-based and metallic particle-based systems), biolistic injection, and the like. As used herein, the term "viral gene transfer system" refers to gene transfer systems comprising viral elements (e.g., intact viruses, modified viruses and viral components such as nucleic acids or proteins) to facilitate delivery of a sample (e.g., a nucleic acid encoding a fusion protein of the present invention) to a desired cell or tissue. As used herein, the term "adenovirus gene transfer system" refers to gene transfer systems comprising intact or altered viruses belonging to the family Adenoviridae.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl)uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "gene" refers to a nucleic acid (e.g., DNA) sequence that comprises coding sequences necessary for the production of a polypeptide, RNA (e.g., including but not limited to, mRNA, tRNA and rRNA) or precursor. The polypeptide, RNA, or precursor can be encoded by a full length coding sequence or by any portion thereof. The term also encompasses the coding region of a structural gene and the sequences located adjacent to the coding region on both the 5' and 3' ends for a distance of about 1 kb on either end such that the gene corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In particular, the terms "target protein gene" or "target protein genes" refer to the full-length target protein sequence. However, it is also intended that the term encompass fragments of the target protein sequences, mutants of the target protein sequences, as well as other domains within the full-length target protein nucleotide sequences. Furthermore, the terms "target protein nucleotide sequence" or "target protein polynucleotide sequence" encompasses DNA, cDNA, and RNA (e.g., mRNA) sequences.

Where "amino acid sequence" is recited herein to refer to an amino acid sequence of a naturally occurring protein molecule, "amino acid sequence" and like terms, such as "polypeptide" or "protein" are not meant to limit the amino acid sequence to the complete, native amino acid sequence associated with the recited protein molecule.

The term "wild-type" refers to a gene or gene product that has the characteristics of that gene or gene product when isolated from a naturally occurring source. A wild-type gene is that which is most frequently observed in a population and is thus arbitrarily designed the "normal" or "wild-type" form of the gene. In contrast, the terms "modified," "mutant," "polymorphism," and "variant" refer to a gene or gene product that displays modifications in sequence and/or functional properties (i.e., altered characteristics) when compared to the wild-type gene or gene product. It is noted that naturally-occurring mutants can be isolated; these are identified by the fact that they have altered characteristics (e.g., increased or decreased solubility) when compared to the wild-type gene or gene product.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the terms "nucleotide sequence encoding a gene" and "polynucleotide having a nucleotide sequence encoding a gene," means a nucleic acid sequence comprising the coding region of a gene or, in other words, the nucleic acid sequence that encodes a gene product. The coding region may be present in a cDNA, genomic DNA, or RNA form. When present in a DNA form, the oligonucleotide or polynucleotide may be single-stranded (i.e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors of the present invention may contain endogenous enhancers/promoters, splice junctions, intervening sequences, polyadenylation signals, etc. or a combination of both endogenous and exogenous control elements.

The term "homology" refers to a degree of complementarity. There may be partial homology or complete homology (i.e., identity). A partially complementary sequence is one that at least partially inhibits a completely complementary sequence from hybridizing to a target nucleic acid and is referred to using the functional term "substantially homologous." The term "inhibition of binding," when used in reference to nucleic acid binding, refers to inhibition of binding caused by competition of homologous sequences for binding to a target sequence. The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (i.e., the hybridization) of a completely homologous to a target under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target that lacks even a partial degree of complementarity (e.g., less than about 30% identity); in the absence of non-specific binding the probe will not hybridize to the second non-complementary target.

As used herein, the term "recombinant DNA molecule" as used herein refers to a DNA molecule that is comprised of segments of DNA joined together by means of molecular biological techniques.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced. The present invention is not limited to naturally occurring protein molecules. For example, the present invention contemplates synthesis of fusion proteins comprising multiple regions of unique polypeptide sequences (e.g., a Tat leader sequence, a target protein sequence, and marker protein sequence).

The term "isolated" when used in relation to a protein, as in "an isolated protein" or refers to a protein that is identified and separated from at least one component or contaminant with which it is ordinarily associated in its natural source.

As used herein, the term "purified" or "to purify" refers to the removal of components (e.g., contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

As used herein, the term "vector" is used in reference to nucleic acid molecules that transfer DNA segment(s) from one cell to another. The term "vehicle" is sometimes used interchangeably with "vector." Vectors are often derived from plasmids, bacteriophages, or plant or animal viruses.

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "calcium phosphate co-precipitation" refers to a technique for the introduction of nucleic acids into a cell. The uptake of nucleic acids by cells is enhanced when the nucleic acid is presented as a calcium phosphate-nucleic acid co-precipitate. The original technique of Graham and van der Eb (Graham and van der Eb, Virol., 52:456 (1973)), has been modified by several groups to optimize conditions for particular types of cells. The art is well aware of these numerous modifications.

As used herein, the term "cell culture" refers to any in vitro culture of cells. Included within this term are continuous cell lines (e.g., with an immortal phenotype), primary cell cultures, transformed cell lines, finite cell lines (e.g., non-transformed cells), and any other cell population maintained in vitro.

As used herein, the term "in vitro" refers to an artificial environment and to processes or reactions that occur within an artificial environment. In vitro environments can consist of, but are not limited to, test tubes and cell culture. The term "in vivo" refers to the natural environment (e.g., an animal or a cell) and to processes or reaction that occur within a natural environment.

As used herein, the term "host cell" refers to any cell, whether located in vitro or in vivo, that can be, or has been, a recipient for or incorporates exogenous nucleic acid sequences (e.g., vectors comprising fusion protein sequence), polynucleotides and/or proteins of the present invention. It is also meant to include progeny of a single cell, and the progeny may not necessarily be completely identical (e.g., in morphology or in genomic or total DNA complement) to the original parent cell due to natural, accidental, or deliberate mutations. The cells may be eukaryotic or prokaryotic and include, but are not limited to bacterial cells (e.g., E. coli) yeast cells, mammalian cells, avian cells, amphibian cells, plant cells, fish cells, and insect cells).

### Detailed Description of the Invention

The bacterial twin-arginine translocation (Tat) system is unique in its ability to export folded proteins or protein domains across the tightly sealed cytoplasmic membrane. This remarkable feat is accomplished by a translocase composed of the TatABC integral membrane proteins that function independently of soluble factors or nucleoside triphosphates (Bogsch EG, et al. (1998) JBiol Chem 273(29):18003-18006; Sargent F, et al. (1998) EMBO J 17(13):3640-3650; Settles AM, et al. (1997) Science 278(5342):1467-1470; Weiner JH, et al. (1998) Cell 93(1):93-101). The Tat system appears to accommodate at least two broad classes ofproteins: globular proteins that fold too rapidly to be handled by the well characterized Sec export pathway and proteins that assemble cofactors or protein subunits in the cytoplasm and necessarily must be exported in a folded form (Berks BC (1996) Mol Microbiol 22(3):393-404; Rodrigue et al., (1999) J Biol Chem 274(19):13223-13228; Santini CL, et al. (1998) EMBO J 17(1):101-112). The ability of the Tat pathway to accept these folded substrates has significant implications for the export mechanism and raises key questions about the structure/function of the translocase and whether substrates need to be correctly folded prior to export.

While recent reports suggest that the bacterial Tat machinery can export certain unfolded protein domains (Cline K & McCaffery (2007) EMBO J 26(13):3039-3049; Richter et al., JBiol Chem 282(46):33257-33264), the vast majority of Tat substrates that normally undergo folding in the cytosol are only competent for Tat export if they are correctly folded (DeLisa et al., (2003) Proc Natl Acad Sci USA 100(10):6115-6120; Sanders et al., (2001) Mol Microbiol 41(1):241-246; Matos et al., (2008) EMBO J 27(15):2055-29 2063; Fisher et al., (2006) Protein Sci 15(3):449-458; Lim HK, et al. (2009) Protein Sci 18(12):2537-2549). The present invention is not limited to a particular mechanism. Indeed, an understanding of the mechanism is not necessary to practice the present invention. Nonetheless, it has been speculated that an inbuilt feature of the Tat system is a quality control mechanism that discriminates between folded and unfolded proteins, allowing the export of only the former. In support of this hypothesis, a model Tat substrate composed of the *Escherichia coli* trimethylamine *N*-oxide reductase signal peptide fused to *E. coli* alkaline phosphatase (ssTorA20PhoA) was found to associate with the Tat translocase even when the PhoA moiety was reduced and therefore misfolded (Panahandeh et al., (2008) J Biol Chem 283(48):33267-33275; Richter S & Bruser (2005) J Biol Chem 280(52):42723-42730). Moreover, binding of reduced ssTorA-PhoA to the TatBC receptor site was perturbed compared to its oxidized (e.g., folded) counterpart (Panahandeh et al., supra), indicating some degree of quality control by TatBC. More recently, removal of just 33 C-terminal residues from ssTorA-PhoA was reported to completely block translocation even though PhoA remained oxidized and presumably folded (Maurer et al., (2009) FEBS Lett 583(17):2849-2853), indicating that the Tat machinery even scrutinizes incomplete folding states. Along similar lines, incorrectly folded FeS substrate proteins with mutations in a single FeS cluster were completely blocked for Tat export, and the Tat apparatus was found to directly initiate the degradation of the rejected molecules (Matos et al., supra). Collectively, these findings support a model in which the Tat system is at the center of an integrated quality control system that involves sensing the degree of folding of its protein substrates before transport and also initiating degradation of those that are incompletely folded or assembled. Such substrate quality control appears to involve productive interactions between the substrate and the TatBC components (Panahandeh et al., supra) and indicates that membrane targeting, quality control, and translocation of Tat substrates are distinct steps that can be analyzed separately from each other.

A hallmark of the bacterial twin-arginine translocation (Tat) pathway is its ability to export folded proteins. The present inventors discovered that over-expressed Tat substrate proteins form two distinct, long-lived translocation intermediates that are readily detected by immunolabeling methods. Formation of the early translocation intermediate, Ti-1, which exposes the N- and C-termini to the cytoplasm, did not require an intact Tat translocase, a functional Tat signal peptide, or a correctly folded substrate. In contrast, formation of the later translocation intermediate, Ti-2, which exhibits a bitopic topology with the N-terminus in the cytoplasm and C-terminus in the periplasm, was much more particular, requiring an intact translocase, a functional signal peptide, and a correctly folded substrate protein. The present invention exploits the ability to directly detect Ti-2 intermediates for a new protein engineering technology called MAD-TRAP (membrane-anchored display for Tat-based recognition of associating proteins). This approach enables isolation of properly folded, ligand-binding proteins from combinatorial libraries displayed as Ti-2 intermediates on the periplasmic face of the *Escherichia coli* inner membrane. Using just two rounds of mutagenesis and screening with MAD-TRAP, the intracellular folding and antigen-binding activity of a human single-chain antibody fragment were simultaneously improved. This approach has several advantages for library screening, including the unique involvement of the Tat folding quality control mechanism that ensures only native-like proteins are displayed, thus eliminating poorly folded sequences from the screening process.

The inventors dissected the Tat transport process into several discrete steps that are characterized by distinct translocation intermediates. Previous work on the plant thylakoidal Tat system identified two Tat translocation intermediates (Berghofer & Klosgen (1999) FEBS Lett 460(2):328-332; Hou et al., (2006) J Mol Biol 355(5):957-967). The first was an early translocation intermediate called Ti-1 that was observed to insert into the membrane in a loop-like conformation with both the N- and C-termini exposed to the chloroplast stroma (the cytoplasm equivalent of chloroplasts). In later stages of the transport process, the C-terminal domain of the substrate was translocated across the thylakoid membrane, resulting in the appearance of translocation intermediate-2 (Ti-2) that exhibited a bitopic topology with the N-terminus facing the stroma and the C-terminus in the lumen (the periplasm equivalent). Here, the inventors have identified for the first time similar translocation intermediates in *E. coli* and demonstrate that formation of Ti-2 but not Ti-1 is dependent upon a functional signal peptide, an intact Tat translocase, and correct folding of the substrate. The formation and detection of the Ti-2 intermediate can be exploited for engineering the properties (e.g., folding, binding activity) of ligand-binding proteins such as human single-chain variable fragment (scFv) antibodies.

Accordingly, the present invention provides methods, systems and reagents for screening proteins for desired properties, including but not limited to, enhanced solubility, enhanced intracellular folding efficiency, and enhanced binding affinity for binding partners of the protein, and combinations thereof. In preferred embodiments, the present invention provides methods, systems and reagents for introducing mutations into a target protein to provide a library of mutated target proteins and screening the library of mutated target proteins for mutants with desired properties. In some preferred embodiments, the mutations are introduced into the target protein by specifically or randomly mutating a nucleic acid encoding the target protein. In preferred embodiments, the mutated nucleic acids are expressed in a host cell as a fusion protein comprising a Tat signal sequence operably linked to the N-terminus of the mutated target protein. As described above, this system takes advantage of the observation that expression of the Ti-2 intermediate requires a functional signal peptide, an intact Tat translocase, and correct folding of the substrate. When these conditions are met, the mutated target protein is displayed on the periplasmic surface of the inner membrane. Spheroplasts can then be made from the host cells and screened for binding to a binding partner of the target protein or other desired properties. The present invention can be utilized characterize or monitor the solubility, folding and/or binding or other properties of any protein, and the ability of other factors (e.g., small molecules, pharmaceuticals, etc.) to alter (e.g., enhance or inhibit) these properties of the target protein.

The subject-matter of the present invention as defined in claims 1 and 14 differs from the prior art WO-A-2007/016591, WO-A-2007/024877, Lutz et al., (2002) Protein Engineering 15(12): 1025-1030 and Fisher et al., (2008) Microbial Biotechnology 1(5): 403-415 in the method of screening mutants of a target protein comprising the step of: displaying a library comprising a plurality of host cells; forming spheroplasts from the host cells; contacting spheroplasts with a target protein binding partner; and selecting spheroplasts that bind the target protein. The subject-matter of the present invention as defined in claim 20 differs from said prior art in the library of spheroplasts: The spheroplasts comprising a library of target nucleic acid molecules encoding a fusion proteins comprising a Tat signal sequence operable linked to a mutated target protein so that the fusion proteins adisplayed on the inner membrane of said spheroplasts.

The invention is set out in the appended claims. The embodiments of the description which do not fall within the scope of said claims are provided for illustrative purposes only and do not form part of the present invention.

Methods, systems and reagents are described in more detail below.

### A. Nucleic Acid Sequences and Vectors

### 1. Fusion Proteins

The present invention utilizes fusion protein comprising a Tat signal sequence linked to the N-terminus of a target protein. The target protein may have the same length or amino acid sequence as the endogenously produced protein, if such protein exists. In other embodiments, the target protein may be a truncated protein, protein domain or protein fragment of a larger peptide chain. For example, the target protein may comprise a fragment of an antibody or a membrane embedded or otherwise hydrophobic protein.

In some embodiments, fusion proteins are produced by operatively linking at least one nucleic acid encoding at least one amino acid sequence (e.g., a Tat signal sequence) to at least a second nucleic acid encoding at least a second amino acid sequence (e.g., a target protein amino acid sequence), so that the encoded sequences are translated as a contiguous amino acid sequence either in vitro or in vivo. Fusion protein design and expression is well known in the art, and methods of fusion protein expression are described herein, and in references, such as, for example, U.S. Pat. No. 5,935,824. In some embodiments, linkers are used to join the various portions of the fusion protein. One such linker is another peptide, such as described in U.S. Pat. No. 5,990,275.

In some embodiments, the fusion protein, and nucleic acids encoding the same, comprises a Tat signal sequence operably to a target protein sequence. The present invention is not limited to the use of any particular Tat signal sequence. Suitable Tat signal sequences include, but are not limited to, TorA, CueO, DmsA, FdnG, FdoG, HyaA, NapA, Sufl, TorA, WcaM, YagT, YcbK, YcdB, YdhX, and YnfE Tat signal sequences.

In some embodiments, the fusion proteins (and nucleic acids encoding the fusion proteins) comprise a protein tag, preferably an epitope tag, on the C-terminus of the target protein. Preferably, protein tags are polypeptide sequences that bind to a compound or another protein so that isolation of the tagged fusion protein is facilitated. Suitable protein tags include, but are not limited to, glutathione-S-transferase (GST), the His-tag (e.g., a polyhistidine tag of 5, 6, or 7 histidine residues), the maltose binding protein-tag, SBP-tag, and epitope tags such as the Flag-tag (e.g., N-DYKDDDDK-C), the HA-tag, the Myc-tag, and the like. In these embodiments, the protein tag is the first member of a specific binding pair. The protein tag is preferably detected with a labelled reagent specific for the protein tag, e.g., glutathione for GST, Ni for a His-tag, antibodies for a FLAG-tag, antibodies for the HA-tag, antibodies for the myc-tag, amylase for the MPB-tag, streptavidin for the SBP-tag, etc.

Likewise, the present invention is not limited to the use of any particular target protein. For example, in some embodiments, a target protein may be a wild-type (e.g., full length) protein or may be a peptide fragment thereof (e.g., a polypeptide sequence of 4 or more amino acids, or preferably 10 or more amino acids). In some embodiments, the polypeptides are "heterologous," meaning that they are foreign to the host cell being utilized (e.g., a human protein produced by an E. coli cell, or a mammalian polypeptide produced by a yeast cell, or a human polypeptide produced from a human cell line that is not the native source of the polypeptide). Thus, the target protein may be any protein of interest for which properties such as binding affinity, solubility and/or folding is to be analyzed. For example, the target protein may be Alzheimer's amyloid peptide (Aβ), SOD1, presenillin 1 and 2, renin, α-synuclein, amyloid A, amyloid P, activin, anti-HER-2, bombesin, enkephalinase, protease inhibitors, therapeutic enzymes, α1-antitrypsin, mammalian trypsin inhibitor, mammalian pancreatic trypsin inhibitor, calcitonin, cardiac hypertrophy factor, cardiotrophins (such as cardiotrophin-1), CD proteins (such as CD-3, CD-4, CD-8 and CD-19), CFTR, CTNF, DNase, human chorionic gonadotropin, mouse gonadotropin-associated peptide, cytokines, transthyretin, amylin, lipoproteins, lymphokines, lysozyme, a growth hormone (including human growth hormone), bovine growth hormone, growth hormone releasing factor, parathyroid hormone, thyroid stimulating hormone, growth factors, brain-derived neurotrophic growth factor, epidermal growth factor (EGF), fibroblast growth factor (such as α FGF and β FGF), insulin-like growth factor-I and -II, des(1-3)-IGF-I (brain IGF-I), insulin-like growth factor binding proteins, nerve growth factor (such as NGF-β), platelet-derived growth factor (PDGF), vascular endothelial growth factor (VEGF), receptors for growth hormones or growth factors, transforming growth factor (TGF) (such as TGF-α, TGF-β1, TGF-β2, TGF-β3, TGF-β4 or TGF-β5), neurotrophic factors (such as neurotrophin-3, -4 ,-5, or -6), gelsolin, glucagon, kallikreins, mullerian-inhibiting substance, neurotrophic factors, p53, protein A or D, prorelaxin, relaxin A-chain, relaxin B-chain, rheumatoid factors, rhodopsin, a serum albumin (such as human serum albumin), inhibin, insulin, insulin chains, insulin A-chain, insulin β-chain, insulin receptor, proinsulin, luteinizing hormone, integrin, interleukins (ILs) (such as IL-1 to IL-10, IL12, IL-13), erythropoietin, thrombopoietin, fibrillin, follicle stimulating hormone, clotting factors (such as factor VIIIC, factor IX, tissue factor, and von Willebrands factor, anti-clotting factors (such as Protein C, atrial naturietic factor, lung surfactant), a plasminogen activator (such as human tissue plasminogen activator or urokinase), thrombin, tumor necrosis factor-α or β, α-ketoacid dehydrogenase, addressins, bone morphogenetic proteins (BMPs), collagen, colony stimulating factors (CSFs) (such as M-CSF, GM-CSF and G-CSF), decay accelerating factor, homing receptors, interferons (such as interferon-α, -β and -y), keratin, osteoinductive factors, PRNP, regulatory proteins, superoxide dismutase, surface membrane proteins, transport proteins, T-cell receptors, viral antigens such as a portion of the AIDS envelope, immunoglobulin light chain, antibodies, antibody fragments (such as single-chain Fv fragment (scFv), single-chain antibody (scAb), F_{AB} antibody fragment, diabody, triabody, fluorobody), antigens such as gp120(IIIb) immunotoxins, atrial natriuretic peptide, seminal vesicle exocrine protein, β2-microglobulin, PrP, precalcitonin, ataxin 1, ataxin 2, ataxin 3, ataxin 6, ataxin 7, huntingtin, androgen receptor, CREB-binding protein, gp120, p300, CREB, AP1, ras, NFAT, jun, fos, dentaorubral pallidoluysian atrophy-associated protein, a microbial protein (e.g., maltose binding protein, ABC transporter, glutathione S transferase, thioredoxin, β-lactamase), green fluorescent protein, red fluorescent protein, or derivatives or active fragments or genetic variants of any of the peptides listed above. The polypeptides may be native or mutated polypeptides, and preferred sources for such mammalian polypeptides include human, bovine, equine, porcine, lupine and rodent sources, with human proteins being particularly preferred.

In some preferred embodiments, the target protein is an antigen binding molecule, such an antibody or antibody fragment. The present invention is not limited by the type of antibody or antibody fragment. Indeed, a variety of antibodies or antibody fragments may be used in the compositions and methods of the present invention including, but not limited to, all varieties of single chain antibody fragments (e.g., Fab, Fab₂ (bispecific), Fab₃ (trispecific) scAb, scFv, Bis-scFv, Diabody, Triabody, Minibody, Tetrabody, Transbody, ADEPT molecule (scFv-enzyme fusion), immunotoxin, VhH domain, V-NAR domain, V_{H} domain, VL domain, Camel Ig, IgNAR, and IgG). In some embodiments, the antibody is an intrabody, i.e., an antibody that acts within the cell or is directed to an intracellular epitope.

In addition, the target protein may be selected from the group comprising single chain T cell receptor ligands (scTCRs); recombinant T cell receptor ligands (RTLs); single-chain class I and II MHC molecules; non-antibody binding proteins (e.g., fluorobodies, peptide aptamers, Affibody, Maxibody, Tetranectin (e.g., C-type lectin), IMabs, AdNectin, Kunitz-type domain from human or bovine trypsin inhibitor, Evibody, ankyrin repeat protein, anticalin (e.g., human lipocalin), affilin molecule (e.g., human gamma-crystallin/human ubiquitin), and Microbody.

In some embodiments, the target protein is a hormone receptor (e.g., a nuclear hormone receptor) or a ligand for a nuclear hormone receptor. Nuclear hormone receptors are grouped into a large superfamily and are thought to be evolutionarily derived from a common ancestor. Seven subfamilies of mammalian nuclear receptors exist. Class I comprises thyroid hormone receptor, retinoic acid receptor, vitamin D receptor, peroxisome proliferator activated receptor, pregnane X receptor, constitutive androstane receptor, liver X receptor, farnesoid X receptor, reverse ErbA, retinoid Z receptor/retinoic acid-related orphan receptor and the ubiquitous receptor. Class II comprises retinoid X receptor, chicken ovalbumin upstream promoter transcription factor, hepatocyte nuclear factor 4, tailles-related receptor, photoreceptor-specific nuclear receptor and testis receptor. Class III comprises glucocorticoid receptor, androgen receptor, progesterone receptor, estrogen receptor and estrogen-related receptor. NGF-induced clone B is a class IV nuclear receptor; steroidogenic factor 1 and Fushi Tarazu factor 1 are class V receptors; germ cell nuclear factor is a class VI receptor; and, small heterodimeric partner and dosage-sensitive sex reversal are class 0 receptors (See, e.g., Aranda and Pascual, Physiol Rev. 2001, 81(3):1269-1304).

Ligands for some of these types of receptors have been identified, for example, products of lipid metabolism such as fatty acids, prostaglandins, or cholesterol derivatives have been shown to regulate gene expression by binding to nuclear receptors. These nuclear receptors bind to hormone response elements as monomers, homodimers, or RXR heterodimers. Ligands may play a role in dimerization and binding to DNA (See, e.g., Ribeiro, Kidney Int. 1992, 42(6):1470-83). A number of proteins interact with these receptors, including general transcription factors. As with other transcriptional regulatory proteins, one aspect of the mechanisms by which nuclear receptors affect the rate of RNA polymerase II-directed transcription likely involves the interaction of receptors with components of the transcription preinitiation complex. This interaction may be direct, or it may occur indirectly through the action of bridging factors (See, e.g., Schulman, Curr Opin Neurobiol. 1995, (3):375-81). Sequence-specific transcription factors, coactivators and corepressors (See, e.g., Cavailles et al., 1995, EMBO J. 1995 Aug 1;14(15):3741-51) also have been found to interact with these nuclear receptors. Thus, in some embodiments, compositions and methods of the present invention are useful for analysis of nuclear hormone receptors and their ligands.

In some embodiments, compositions and methods of the present invention are used to identify agents (e.g., test compounds/candidate compounds) that alter (e.g., enhance or inhibit) ligand binding to a receptor, such as a growth factor receptor or hormone receptor.

The polynucleotides and sequences embodied in this invention can be obtained using, among other methods, chemical synthesis, recombinant cloning methods, PCR, or any combination thereof. PCR technology is the subject matter of U.S. Pat. Nos. 4,683,195; 4,800,159; 4,754,065; and 4,683,202 and described in PCR: THE POLYMERASE CHAIN REACTION (Mullis et al. eds, Birkhauser Press, Boston (1994)) and references cited therein. Alternatively, one of skill in the art can use the sequences provided herein, or available from other sources (e.g., ncbi.nlm.nih.gov) and a commercial DNA synthesizer, PCR, or other molecular biological techniques to synthesize or otherwise attain the nucleic acid sequence (e.g., DNA sequence) of any target protein of interest.

Once the target protein of interest and Tat signal sequence are chosen, they may be operatively expressed in a recombinant vector. The vector may be expressed in vitro or in vivo for analyzing and/or altering target protein solubility and/or folding. As used herein, the term "vector" is used in reference to nucleic acid molecules that transfer nucleic acid (e.g., DNA) segment(s) from one cell to another. The term "vehicle" is sometimes used interchangeably with "vector." A nucleic acid sequence can be "exogenous" or "heterologous," which means that it is foreign to the cell into which the vector is being introduced or that the sequence is homologous to a sequence in the cell but in a position within the host cell nucleic acid in which the sequence

is ordinarily not found. Vectors include, but are not limited to, plasmids, cosmids, viruses (bacteriophage, animal viruses, and plant viruses), and artificial chromosomes (e.g., YACs). One of skill in the art would be well equipped to construct a vector through standard recombinant techniques, which are described in Sambrook et al., 1989 and Ausubel et al., 1994.

The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals. In addition to control sequences that govern transcription and translation, vectors and expression vectors may contain nucleic acid sequences that serve other functions as well, some of which are described below.

In preferred embodiments, the vectors of the present invention comprise in 5' to 3' order or other operable order a Tat signal sequence, a multiple cloning site, and target protein sequence. In preferred embodiments, the protein of interest is inserted into the multiple cloning site. The sequence of the protein of interest can also be easily removed for cloning into other vectors for use in other assay of screening steps, such as iterative directed evolution procedures or use in other protein folding assays. In preferred embodiments, the vector is a plasmid containing additional elements useful expressing the fusion protein in a host cell, such as a preferred *E. coli* strain. In further preferred embodiments, the vectors additional comprise one or more of the elements described below.

A "promoter" is a control sequence that is a region of a nucleic acid sequence at which initiation and rate of transcription are controlled. It may contain genetic elements at which regulatory proteins and molecules may bind such as RNA polymerase and other transcription factors. The phrases "operatively positioned," "operatively linked," "under control," and "under transcriptional control" mean that a promoter is in a correct functional location and/or orientation in relation to a nucleic acid sequence (e.g., a nucleic acid sequence encoding a fusion protein of the present invention) to control transcriptional initiation and/or expression of that sequence. A

promoter may or may not be used in conjunction with an "enhancer," which refers to a cis-acting regulatory sequence involved in the transcriptional activation of a nucleic acid sequence.

A promoter may be one naturally associated with a gene or sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally occurring," e.g., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR, in connection with the compositions disclosed herein (see U.S. Pat. No. 4,683,202, U.S. Pat. No. 5,928,906). It is further contemplated that control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment (e.g., comprising nucleic acid encoding a fusion protein of the present invention) in the cell type, organelle, and organism chosen for expression. Those of skill in the art of microbiology and molecular biology generally know the use of promoters, enhancers, and cell type combinations for protein expression, for example, see Sambrook et al. (1989). The promoters employed may be constitutive, tissue-specific, inducible, and/or useful under the appropriate conditions to direct the desired level expression of the introduced DNA segment comprising a target protein of the present invention (e.g., high levels of expression that are advantageous in the large-scale

production of recombinant proteins and/or peptides). The promoter may be heterologous or endogenous.

Multiple elements/promoters may be employed in the context of the present invention to regulate the expression of nucleic acid encoding a fusion protein of the present invention. For example, the promoter/element may be, but is not limited to, lac, pho (e.g. phoA), tac, trc, trp, tet, araBAD, P_{L} T3, T7, T7-lac and SP6. Furthermore, it is contemplated that any inducible or constitutively active promoter finds use in the present invention.

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picomavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Pat. Nos. 5,925,565 and 5,935,819).

Vectors may include a multiple cloning site (MCS), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant nucleic acid technology.

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression.

In expression, a polyadenylation signal may be included to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Also contemplated as an element of the expression cassette is a transcriptional termination site. These elements can serve to enhance message levels and/or to minimize read through from the cassette into other sequences.

In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

In certain embodiments of the invention, in addition to the portion of the fusion protein, and nucleic acid sequences encoding the same, that contains a marker protein, a cell that contains a fusion protein nucleic acid construct of the present invention may be identified in vitro or in vivo by including a marker (e.g., either the same or different marker than that present in the fusion protein) in the expression vector. Such markers confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker.

The inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes such as herpes simplex virus thymidine kinase (tk) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a fusion protein of the present invention. Further examples of selectable and screenable markers are well known to one of skill in the art.

### 2. Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a prokaryotic or eukaryotic cell, and it includes any transformable organisms that is capable of replicating a vector and/or expressing a heterologous gene encoded by a vector. In some embodiments, a host cell is used as a recipient for vectors. A host cell may be "transfected" or "transformed," which refers to a process by which exogenous nucleic acid is transferred or introduced into the host cell. A transformed cell includes the primary subject cell and its progeny.

The fusion protein constructs, host cells and methods of the present invention are also useful for identifying variations in a process for biosynthesis of a target protein. The process can be varied to modify the solubility of the target protein. For example, a cell containing a fusion protein nucleic acid is cultured under alternative conditions and the growth of the host cells under selective conditions monitored. For example, protein solubility may be affected by the temperature, medium composition, or oxygen concentration in which the host cells are cultured. The method by which host cell growth is measured provides an immediate readout of solubility and permits a variety of alternative conditions to be tested with minimal effort, to identify those conditions where the highest proportion of soluble target protein is produced.

As used herein, the terms "engineered" and "recombinant" cells or host cells are intended to refer to a cell into which an exogenous DNA segment or gene, such as a cDNA or gene encoding at least one fusion protein has been introduced. Therefore, engineered cells are distinguishable from naturally occurring cells which do not contain a recombinantly introduced exogenous DNA segment or gene. Engineered cells are thus cells having a gene or genes introduced through human intervention. Recombinant cells include those having an introduced cDNA or genomic gene, and also include genes positioned adjacent to a promoter not naturally associated with the particular introduced gene.

The invention is not limited to any particular host cell. A host cell may be prokaryotic or eukaryotic. In some embodiments, prokaryotic host cells are *E. coli* strain MC4100, B1LK0, RR1, E. coli LE392, E. coli B, E. coli X 1776 (ATCC No. 31537) as well as E. coli W3110 (F-, prototrophic, ATCC No. 273325); bacilli such as Bacillus subtilis; and other enterobacteriaceae such as Salmonella typhimurium, Serratia marcescens, and various Pseudomonas species. However, potential host cells are not limited to these examples. Indeed, a host cell may be any species of bacteria selected from the group consisting of Acetobacter, Actinomyces, Aerobacter, Agribacterium, Azotobacter, Bacillus, Bacteroides, Bordetella, Brucella, Chlamydia, Clostridium, Corynebacterium, Erysipelothrix, Escherichia, Francisella, Fusobacterium, Haemophilus, Klebsiella, Lactobacillus, Listeria, Mycobacterium, Myxococcus, Neisseria, Nocardia, Pasteurella, Proteus, Pseudomonas, Rhizobium, Rickettsia, Salmonella, Serratia, Shigella, Spirilla, Spirillum, Staphylococcus, Streptococcus, Streptomyces, Trepanema, Vibrio, Vibrio, and Yersinia. Alternatively, the host cells may be mammalian cells such as CHO cells.

With regard to the expression of fusion proteins of the present invention, once a suitable fusion protein nucleic acid encoding sequence has been obtained, one may proceed to prepare an expression system (e.g., expressing fusion protein constructs within host cells). The engineering of DNA segment(s) for expression in a prokaryotic or eukaryotic system may be performed by techniques generally known to those of skill in recombinant expression.

It is believed that virtually any expression system may be employed in the expression of the proteins of the present invention. Prokaryote- and/or eukaryote-based systems can be employed for use with the present invention to produce nucleic acid sequences, or their cognate polypeptides, proteins and peptides. Many such systems are commercially and widely available.

While it is conceivable that a fusion protein may be delivered directly, a preferred embodiment involves introducing a nucleic acid encoding a fusion protein of the present invention to a cell. Following introduction into the host cell, the fusion protein is synthesized by the transcriptional and translational machinery of the cell. In some embodiments, additional components useful for transcription or translation may be provided by the expression construct comprising fusion protein nucleic acid sequence.

In some embodiments, the nucleic acid encoding the fusion protein may be stably integrated into the genome of the cell. In yet further embodiments, the nucleic acid may be stably maintained in the cell as a separate, episomal segment of DNA, such as a plasmid. Such nucleic acid segments or "episomes" encode sequences sufficient to permit maintenance and replication independent of or in synchronization with the host cell cycle. How the expression construct is delivered to a cell and where in the cell the nucleic acid remains is dependent on, among other things, the type of expression construct employed.

A number of procedures exist for the preparation of competent bacteria and the introduction of DNA into those bacteria. Protocols for the production of competent bacteria have been described (Hanahan (J. Mol. Biol. 166: 557-580 (1983); Liu et al., Bio Techniques 8:21-25 (1990); Kushner, In: Genetic Engineering: Proceedings of the International Symposium on Genetic Engineering, Elsevier, Amsterdam, pp. 17-23 (1978); Norgard et al., Gene 3:279-292 (1978); Jessee et al., U.S. Pat. No. 4,981,797); Maniatis et al., Molecular Cloning, A Laboratory Manual, Cold Spring Harbor Press, Cold Spring Harbor, N.Y. (1982).

Another rapid and simple method for introducing genetic material into bacteria is electroporation (Potter, Anal. Biochem. 174: 361-73 (1988)). This technique is based upon the original observation by Zimmerman et al., J. Membr. Biol. 67: 165-82 (1983), that high-voltage electric pulses can induce cell plasma membranes to fuse. Subsequently, it was found that when subjected to electric shock (typically a brief exposure to a voltage gradient of 4000-16000 V/cm), the bacteria take up exogenous DNA from the suspending solution, apparently through holes momentarily created in the plasma membrane. A proportion of these bacteria become stably transformed and can be selected if a suitable marker gene is carried on the transforming DNA (Newman et al., Mol. Gen. Genetics 197: 195-204 (1982)). With E. coli, electroporation has been found to give plasmid transformation efficiencies of 10.sup.9-10.sup.10 T/ug DNA (Dower et al., Nucleic Acids Res. 16: 6127-6145 (1988)).

Bacterial cells are also susceptible to transformation by liposomes (Old and Primrose, In: Principles of Gene Manipulation: An Introduction to Gene Manipulation, Blackwell Science (1995)). A simple transformation system has been developed which makes use of liposomes prepared from cationic lipid (Old and Primrose, In: Principles of Gene Manipulation: An Introduction to Gene Manipulation, Blackwell Science (1995)). Small unilamellar (single bilayer) vesicles are produced. DNA in solution spontaneously and efficiently complexes with these liposomes (in contrast to previously employed liposome encapsidation procedures involving non-ionic lipids). The positively-charged liposomes not only complex with DNA, but also bind to bacteria and are efficient in transforming them, probably by fusion with the cells. The use of liposomes as a transformation or transfection system is called lipofection.

It is contemplated that proteins may be expressed in cell systems or grown in media that enhance protein production. One such system is described in U.S. Pat. No. 5,834,249. In certain embodiments, the fusion protein may be co-expressed with one or more proteins that enhance refolding. Such proteins that enhance refolding include, for example, DsbA or DsbC proteins. A cell system co-expressing the DsbA or DsbC proteins are described in U.S. Pat. No. 5,639,635. In certain embodiments, it is contemplated that a temperature sensitive expression vector may be used to aid assaying protein folding at lower or higher temperatures than many E. coli cell strain's optimum growth at about 37.degree. C. For example, a temperature sensitive expression vectors and host cells that express proteins at or below 20.degree. C. is described in U.S. Pat. Nos. 5,654,169 and 5,726,039.

In some preferred embodiments, bacterial host cells expressing the fusion proteins of the present invention treated to remove bacterial cell wall to provide spheroplasts. In some preferred embodiments, the host cells are treated with lysozyme by procedures known in the art.

### 3. Screening Systems

The present invention provides systems and methods for screening of host cells comprising libraries of variants of the same protein or different proteins. In some embodiments, the use of panning technologies allows for the high throughput analysis of mutated target proteins. Accordingly, the present invention provides libraries of host cells, in which the cells of each population differ in the fusion protein expressed by the cells. For example, the fusion proteins can differ due to amino acid substitutions, deletions, or insertions in the target protein compared to a reference target protein amino acid sequence (e.g., an unmodified or wild type target protein sequence). Alternatively, the target proteins expressed by the populations of host cells can be different fragments of a larger polypeptide. In some embodiments, each of the host cells expresses one distinct mutated target protein sequence (e.g., the same host cell does not expresses multiple species of target proteins).

In some embodiments, target proteins with a desired binding property are identified by contacting spheroplasts expressing the fusion proteins of the present invention (e.g., spheroplasts expressing and displaying a library of mutagenized target proteins) with a binding partner or ligand of the target protein. In some embodiments, the binding partner or ligand is labelled with a detectable moiety for direct or indirect detection, for example fluorescent or colorimetric detection. Spheroplasts displaying a target protein with a desired binding property can detected, directly or indirectly, via the labelled binding partner and the nucleic acid encoding the target protein can be isolated and cloned. In some embodiments, the labelled spheroplasts are isolated by flow cytometry, fluorescent activated cell sorting, or similar methods.

In other embodiments, the binding partner for the target protein is immobilized on a support medium. Suitable support media include, but are not limited to, magnetic beads, a polymeric beads, planar supports such as plastic or glass slides and tissue culture plates including multiwell plates, and chromatography supports that display the binding partner. The spheroplasts displaying the target protein can be contacted with the support media. Spheroplasts that display target proteins with desired binding properties bind to the immobilized binding partner and can be isolated from or enriched as compared spheroplasts that either display non-binding or weakly binding target proteins or spheroplasts that express a target protein that is incorrectly folded (and thus not displayed) or that are folded inefficiently (and thus are not displayed at high levels). In some embodiments, the spheroplasts that bind to the immobilized binding partner are eluted from the support medium, for example by changing properties of the buffer such as ionic strength. These embodiments thus provide spheroplasts or an enriched population of spheroplasts that display target proteins with a desired property. The nucleic acid encoding the target protein can be isolated and cloned from the selected or enriched spheroplasts. In some embodiments, the nucleic acid is subjected to additional mutagenesis and additional panning steps are performed in an interative process. The process may be repeated as many times as necessary to achieve target proteins with desired properties, for example, the process may be repeated 2, 3, 4, 5, or 10 times or more.

In some embodiments, target proteins with increased affinity for a binding partner (e.g., affinity of an antibody for an epitope or antigen, or affinity of a growth factor for a growth factor receptor) are selected via an interative panning process. This is typically achieved by affinity selection or panning, using a target compound (e.g., an antigen) to which the displayed molecules (e.g., antibodies) are intended to bind. If desired, several rounds of enrichment procedures can be carried out, e.g., under conditions with increasingly higher stringency. In some embodiments, the nucleic acid encoding the target protein displayed on the selected spheroplasts is cloned and subjected to mutagenesis, and the panning process repeated. In some embodiments, the panning process is conducted with a competitive binding compound in addition to the binding partner to increase enrichment for spheroplasts displaying target proteins with increased binding affinities.

In some embodiments, the present invention utilizes two color screens. In these embodiments, the fusion protein preferably comprises a C-terminal protein tag. The protein tag is detected with a fluorescently labelled reagent that specifically binds the protein tag, e.g., labelled anti-FLAG antibody for FLAG or a labelled Ni reagent for the His-Tag. In preferred embodiments, detection of the protein tag is correlated to the expression level of the target protein (i.e., spheroplasts that express a high level of the target protein exhibit a higher amount of fluorescence). In preferred embodiments, high expression levels of the target protein are indicative of enhanced or improved solubility and/or folding of the target protein. In some embodiments, the labelled protein tag reagent and labelled binding partner are used simultaneously. In these embodiments, the degree of labeling with labelled protein tag reagent (e.g., labelled antibody such as a labelled FLAG antibody) is indicative of expression level of the target protein (and in particular folding and/or solubility) and the degree of labeling with the labelled binding partner is indicative of binding activity. This dual labeling is very powerful as it ensures isolation of clones that exhibit improvements in both expression and antigen binding.

Numerous fluorochromes can be used for labelling, and can be selected, for example from Invitrogen, e.g., see, The Handbook--A Guide to Fluorescent Probes and Labeling Technologies, Invitrogen Detection Technologies, Molecular Probes, Eugene, Oreg.). Examples of particular fluorophores that can be attached (for example, chemically conjugated) to a nucleic acid molecule or protein such as an antigen binding molecule include, but are not limited to, 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcouluarin (Coumaran 151); cyanosine; 4',6-diaminidino-2-phenylindole (DAPI); 5',5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate (FITC), and QFITC (XRITC); 2',7'-difluorofluorescein (OREGON GREEN™); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron™ Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives.

Accordingly, in some embodiments, the present invention provides methods for screening an expression library of clones to identify those clones that express a target protein with desired properties such as enhanced solubility, intracellular folding efficiency, binding affinity for said target protein binding partner, and combinations thereof. In preferred embodiments, the library comprises alterations in the gene (or portion thereof) expressing the target protein (or portion thereof) of interest. Alterations of the gene can be provided by any of several widely used methods. These include, but are not limited to, making truncations in the gene, random chemical mutagenesis, random mutagenesis through erroneous nucleotide incorporation, or site-directed mutagenesis methods. This library of alterations can then be transformed into host cells. Individual clones of the transformed host cells are then cultured under conditions where fusion proteins containing a target protein, or altered form thereof, are expressed. The host cells can be screened as described elsewhere herein.

The present invention also provides methods for screening for mutations in a host cell or in a target protein sequence that improve desired properties of a target protein. For example, cells comprising a fusion protein of the present invention can be treated with a mutagen, and those host cells that display an increase in growth (e.g., rate or abundance) in the presence of a selective marker (e.g., ampicillin) identified. A "mutagen" is intended to include, but not be limited to chemical mutagens such as ethyl methane sulphonate, N-methyl-N'-nitroso-guanidine and nitrous acid as well as physical agents such as ionizing radiation.

In some preferred embodiments, mutations can be introduced into a polynucleotide sequence encoding a target protein. The altered polynucleotide is then tested to determine whether a desired property of the target protein is changed. Such mutations include, but are not limited to, mutations induced by a mutagen; site directed mutations that alter specific amino acid residues such as mutation of cysteine residues to eliminate disulfide bonds; deletions that remove sets of specific amino acids such as deletion of a continuous stretch of hydrophobic amino acids; and fusions of the target protein to a second, particularly soluble protein.

Accordingly, the present invention provides methods where mutations are introduced into the nucleic acid sequences of one or more proteins of interest to provide a library of variant or mutagenized target nucleic acid sequences. In some embodiments, directed evolution procedures are used to prepare libraries of nucleic acid sequences in which a target protein of interest has been mutagenized. The mutagenized nucleic acid sequences are preferably cloned into vectors of the present invention behind a Tat signal sequence. The vectors are then introduced into host cells to provide a library of host cells comprising the nucleic acid sequences of interest. The host cells are then made into spheroplasts and screened, for example by panning with a binding partner of the target protein. In some embodiments, the methods provide for selection of antigen binding protein with improved affinity through an affinity maturation process. Clones of the host cells that express target protein with desired properties are identified and grown. The mutagenized target protein of interest can then be identified, for example, by subcloning and subsequent sequencing or just by sequencing.

In some embodiments, variants may be produced by methods such as directed evolution or other techniques for producing combinatorial libraries of variants. The synthesis of degenerate oligonucleotides is well known in the art (See e.g., Narang, Tetrahedron Lett., 39:39 (1983); Itakura et al., Recombinant DNA, in Walton (ed.), Proceedings of the 3rd Cleveland Symposium on Macromolecules, Elsevier, Amsterdam, pp 273-289 (1981); Itakura et al., Annu. Rev. Biochem., 53:323 (1984); Itakura et al., Science 198:1056 (1984); Ike et al., Nucl. Acid Res., 11:477 (1983)). Such techniques have been employed in the directed evolution of proteins (See e.g., Scott et al., Science 249:386 (1980); Roberts et al., Proc. Natl. Acad. Sci. USA 89:2429 (1992); Devlin et al., Science 249: 404 (1990); Cwirla et al., Proc. Natl. Acad. Sci. USA 87: 6378 (1990); as well as U.S. Pat. Nos. 5,223,409, 5,198,346, and 5,096,815). In some preferred embodiments, error prone PCR is used to introduce mutations into the nucleic acid sequence of the target protein of interest.

In some embodiments, the methods described above are used to prescreen large combinatorial libraries of proteins. Accordingly, the screening methods of the present invention may be combined with other screening methods, for example, a protein binding and/or folding screen of the present invention may precede or be interspersed with other screening steps, such as iterative rounds of directed evolution as described in the patents and publications referenced above. In these methods, variant target proteins with desired properties can be removed from the library prior to further screening to decrease the number of clones or variants that need to be screened.

The present invention also contemplates the use of other methods of introducing mutations into nucleic acid sequences. Chemical mutagenesis offers certain advantages, such as the ability to find a full range of mutant alleles with degrees of phenotypic severity, and is facile and inexpensive to perform. The majority of chemical carcinogens produce mutations in DNA. Benzo(a)pyrene, N-acetoxy-2-acetyl aminofluorene and aflotoxin B1 cause GC to TA transversions in bacteria and mammalian cells. Benzo(a)pyrene also can produce base substitutions such as AT to TA. N-nitroso compounds produce GC to AT transitions. Alkylation of the 04 position of thymine induced by exposure to n-nitrosourea results in TA to CG transitions.

In some embodiments, compositions and methods of the present invention can be designed for the identification and/or characterization of genes encoding proteins that physically interact with a protein/drug complex. For example, in some embodiments, if the target protein and binding partner are able to interact in a drug-dependent manner, the interaction may be detected by host cell growth.

Another aspect of the present invention relates to the use of the screening systems in the development of assays that can be used to screen test compounds that are either agonists or antagonists of a protein-protein interaction of therapeutic consequence (See, e.g., U.S. Pat. No. 6,200,759). In a general sense, the assay evaluates the ability of a test compound to modulate (e.g., enhance or inhibit) binding between target proteins and their binding partners.

The present invention is not limited by the type of test compound. In some embodiments, the test compound is one of a library of test compounds. The present invention is not limited by the type of test compound assayed (e.g., to identify and characterize test compounds capable of altering (e.g., enhancing or inhibiting) the interaction between two or more molecules (e.g., peptides or proteins (e.g., the interaction of which is characterized using the compositions and methods of the present invention)). Indeed a variety of test compounds can be analyzed by the present invention including, but not limited to, any chemical entity, pharmaceutical, drug, known and potential therapeutic compounds, small molecule inhibitors, pharmaceuticals, a test compound from a combinatorial library (e.g., a biological library; peptoid library, spatially addressable parallel solid phase or solution phase library; synthetic library (e.g., using deconvolution or affinity chromatography selection)), and the like. Examples of test compounds useful in the present invention include, but are not limited to, carbohydrates, monosaccharides, oligosaccharides, polysaccharides, amino acids, peptides, oligopeptides, polypeptides, proteins, nucleosides, nucleotides, oligonucleotides, polynucleotides, including DNA and DNA fragments, RNA and RNA fragments and the like, lipids, retinoids, steroids, glycopeptides, glycoproteins, antibody and antibody fragments, proteoglycans and the like, and synthetic analogues or derivatives thereof, including peptidomimetics, small molecule organic compounds and the like, and mixtures thereof.

For example, in some embodiments, an assay is designed to identify and/or characterize a test compound's ability to alter (e.g., enhance or inhibit) the interaction of two polypeptide sequences (e.g., proteins) known to interact. In some embodiments, the two polypeptides known to interact are a ligand and a ligand receptor (e.g., a hormone and a hormone receptor, a growth factor and a growth factor receptor, or any other known interaction between two polypeptide (e.g., protein) sequences). In some embodiments, a test compound is identified that can be utilized for treating (e.g., prophylactically and/or therapeutically) a subject.

The test compounds of the present invention can be obtained using any of the numerous approaches in combinatorial library methods known in the art, including biological libraries; peptoid libraries (e.g., libraries of molecules having the functionalities of peptides, but with a novel, non-peptide backbone, which are resistant to enzymatic degradation but which nevertheless remain bioactive; See, e.g., Zuckennann et al., J. Med. Chem. 37: 2678-85 (1994)); spatially addressable parallel solid phase or solution phase libraries; synthetic library methods requiring deconvolution; the 'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (See, e.g., Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 (1993); Erb et al., Proc. Nad. Acad. Sci. USA 91:11422 (1994); Zuckermann et al., J. Med. Chem. 37:2678 (1994); Cho et al., Science 261:1303 (1993); Carrell et al., Angew. Chem. Int. Ed. Engl. 33.2059 (1994); Carell et al., Angew. Chem. Int. Ed. Engl. 33:2061 (1994); and Gallop et al., J. Med. Chem. 37:1233 (1994).

The interaction between two molecules (e.g., a target protein and a binding partner) can also be detected and/or characterized using fluorescence energy transfer (FRET) (See, e.g., Lakowicz et al., U.S. Patent No. 5,631,169; Stavrianopoulos et al., U.S. Patent No. 4,968,103). A fluorophore label is selected such that a first donor molecule's emitted fluorescent energy will be absorbed by a fluorescent label on a second, 'acceptor' molecule, which in turn is able to fluoresce due to the absorbed energy.

In another embodiment, characterizing the ability of a target protein to bind to a binding partner can be accomplished using real-time Biomolecular Interaction Analysis (BIA) (See, e.g., Sjolander and Urbaniczky, Anal. Chem. 63:2338-2345 (1991) and Szabo et al. Curr. Opin. Struct. Biol. 5:699-705 (1995)). "Surface plasmon resonance" or "BIA" detects biospecific interactions in real time, without labeling any of the interactants (e.g., BIACORE). Changes in the mass at the binding surface (e.g., indicative of a binding event) result in alterations of the refractive index of light near the surface (the optical phenomenon of surface plasmon resonance (SPR)), resulting in a detectable signal that can be used as an indication of real-time reactions between biological molecules.

The ability of test compounds to alter (e.g., increase or decrease) specific protein interaction, while concurrently not altering other protein interaction can also be assayed using the compositions and methods of the present invention. For example, in some embodiments, two or more separate combinations of protein-protein interactors can be assayed in the same cell. Screening in this way permits the identification of compounds that can be utilized (e.g., independently, in a pharmaceutical composition, or co-administered) for altering (e.g., enhancing or inhibiting) specific protein interactions while having no harmful effect (e.g., altering interaction) of other interactions.

In some embodiments, test compounds can be solubilized and added to host cells (e.g., in vitro (e.g., in the culture medium). In some embodiments, various concentrations of the test compound are utilized to determine an efficacious dose. In some embodiments, administration of the test compound is consistent over a period of time (e.g., administered one, two or more times a day) so as to keep the concentration of the test compound constant.

Test compounds can be administered in vitro at a variety of concentrations. For example, in some embodiments, test compounds are added to culture medium or to a subject so as to achieve a concentration from about 10 pg/ml to 10 mg/ml, although higher (e.g., greater than 10 mg/ml) and lower (e.g., less than 10 pg/ml) concentrations may also be used.

It is contemplated that a successfully identified test compound (e.g., a test compound, analogue or mimetic identified that is capable of altering (e.g., enhancing or inhibiting) protein interactions can be utilized in a pharmaceutical composition (e.g., to be administered to a subject (e.g., systemically or locally) to alter the protein interaction in the subject (e.g., thereby generating a desired result (e.g., inhibition of receptor stimulation in a cancer patient) in a subject. Thus, the compositions can also be prepared as injectables, either as liquid solutions or suspensions; solid forms suitable for solution in, or suspension in, liquid prior to injection may also be prepared. The compositions of the present invention are often mixed with diluents or excipients which are physiological tolerable and compatible. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH buffering agents.

### Experimental

### Materials and Methods

**Strains and growth conditions.** Wildtype *E. coli* strain MC4100 and its isogenic Δ*tatC* derivative called BILK0 (Bogsch et al., supra) were used for membrane-anchored display of proteins. BL21(DE3) was used for cytoplasmic expression of proteins. Cultures were grown in LB medium supplemented with the appropriate antibiotic, and protein expression was induced with isopropyl β-D-1-thiogalactopyranoside (IPTG, 0.5-1.0 mM) or arabinose (2% w/v) depending on the plasmid used. Antibiotics were supplemented at the following concentrations: ampicillin (100 µg/mL), chloramphenicol (20 µg/mL), and kanamycin (50 µg/mL).

**Spheroplast formation.** Expression of proteins from pSALect-based plasmids was induced overnight at 37°C, and expression of proteins from pBAD18-based plasmids was generally induced for 4 h at 37°C. Following induction, 3 mL of each culture was pelleted in a 1.5 mL microcentrifuge tube. The pellets were washed with 100 µL of ice-cold fractionation buffer (FB; 170.75 M sucrose, 0.1 M Tris buffer pH 8.0) and resuspended in 350 µL of ice-cold FB 18 supplemented with lysozyme (1 mg/mL). While slowly vortexing, 700 µL of EDTA (1 mM, pH 8.0) was added dropwise, and tubes were incubated for 20 min at room temperature. After adding 50 µL of cold MgCl₂ (0.5 M), tubes were incubated on ice for 10 min and then spun down (12000 rcf) for 10 min at 4°C. The supernatant was removed, and the spheroplasts were resuspended in 1 mL of PBS. Spheroplasts were kept on ice until used in subsequent assays.

**Panning with magnetic beads.** Dynabead M-280 tosylactivated beads (Invitrogen) were coated with β-gal for panning scFv libraries. 1 mL of beads (2 x 10⁹ beads) were washed with 0.1 M sodium phosphate buffer (pH 7.4; Buffer 1) and then resuspended in 1 mL of Buffer 1 containing 0.5 mg/mL β-gal. Following overnight incubation at 37°C, beads were washed twice with phosphate buffered saline with 0.1% BSA (w/v) and 2 mM EDTA (pH 7.4; Buffer 2). Beads were resuspended in 1 mL of 0.2 M Tris with 0.1% BSA (pH 8.0; Buffer 3), incubated for 2 h at 37°C to deactivate free tosyl groups, and washed with Buffer 2. Beads were mixed with spheroplasts (prepared as described above) using a ratio of approximately 10:1 cells:beads, with 1 mL total volume in each tube. Binding reactions were incubated with rotation at 4°C for 11 h. After incubation, bead-bound spheroplasts were washed four times with Buffer 2 and resuspended in 25 µL of distilled water in each tube. The DNA for the scFvs displayed on the12 bead-bound spheroplasts was recovered using PCR and then cloned back into the pSALectΔBla cassette. The panning procedure was repeated to enrich for scFvs that bind to β-gal. During screening of the second-generation library, purified scFv clone 1-4 (0.05-50 nM) was included as a competitor to increase the stringency of the screening.

**Plasmid construction.** Native HybO and HybO lacking the C-terminal tail were expressed frompBAD18-HybO-FLAG and pBAD18-HybOΔC-FLAG (Waraho D & DeLisa MP (2009) Proc Natl Acad Sci U S A 106(10):3692-3697). Plasmid pBAD18-Δss-HybO-FLAG and pBAD18-Δss-HybOΔC-FLAG were constructed by amplifying HybO or HybOΔC, respectively, downstream from the signal sequence and including a SacI site in the forward primer and a FLAG tag and XbaI site in the reverse primer. To create artificial Tat-dependent proteins, an expression plasmid for each protein of interest was generated that contained an N-terminal signal peptide derived from E. coli trimethylamine N-oxide reductase (ssTorA). To generate these plasmids, pSALect-ssTorA-MBP-Bla (Fisher et al., (2006) Protein Sci 15(3):449-458) was digested with NdeI and BstBI to remove the MBP and TEM1 β-lactamase (Bla) genes, which resulted in the pSALect-ssTorA cassette plasmid. All genes to be ligated in the pSALect-ssTorA cassette were PCR amplified using primers specific for the target gene that contained an NdeI site in the forward primer and a BstBI site in the reverse primer. For constructs with an N-terminal FLAG epitope tag (DYKDDDDK), the FLAG sequence was included in the forward primer. Likewise, for constructs with a C-terminal FLAG tag, the FLAG sequence was included in the reverse primer.

For constructs with a C-terminal HybO C-tail (HC;AGAIGLLGGVVGLVAGVSVMAV) (Hatzixanthis et al., (2003) Mol Microbiol 49(5):1377-1390), the HC was introduced using two overlapping reverse primers. For the different MBP and scFv13 constructs, template plasmids used for PCR amplification of the target genes were as follows: pSALect-ssTorA-MBP-Bla for the mature domain of maltose binding protein (MBP) lacking its native Sec signal peptide, pHCME31 for the insoluble variant of MBP (G32D, I33P) (Betton J & Hofnung M (1996) J Biol Chem 271(14):8046-8052) also lacking its Sec signal peptide, pPM163 and pPM163-R4 for insoluble scFv13 and soluble variant of scFv13 named scFv13.R4, respectively (Martineau et al., (1998) J Mol Biol 280(1):117-127), pSALect-scFv2610 for anti-Digoxin scFv (laboratory stock), and pBAD18-Cm-ssTorA-scFv-GCN4(λ)-FLAG for the antiGCN4 scFv (Waraho and DeLisa, supra). To generate derivatives that lacked the ssTorA signal peptide, pSALect-ssTorAMBP-Bla was digested with the NotI, which cuts upstream of the ssTorA sequence, and BstBI to remove the ssTorA-MBP-Bla insert. The different MBP and scFv13 constructs described above were then ligated in-frame between the NdeI and BstBI sites of digested pSALect-ssTorA-MBPBla.

The ssTorA(KK) derivatives of the scFv13 and scFv13.R4 were generated using the QuikChange II site-directed mutagenesis kit (Stratagene) to mutate the RR motif in the signal sequence to KK. To generate expression plasmids for MBP constructs with N- terminal FLAG tags in pBAD18-Cm, the pBAD18-Cm plasmid was first digested with SacI and XbaI. Wild-type and mutant MBP genes with and without the HC and with N-terminal FLAG tags were amplified from pSALect constructs with a SacI cut site upstream of ssTorA in the forward primer and with an XbaI site in the reverse primer. A similar approach was used to clone constructs with C-terminal FLAG tags between SacI and PstI of pBAD18-Kan. To express proteins of interest in the cytoplasm, target genes from biopanning experiments were subcloned into a pET21a(+) plasmid with a C-terminal 6x-His tag using an N-terminal NdeI site and a C-terminal NotI site flanking the target gene.

**Combinatorial library construction.** A random mutagenesis library was generated from scFv13 using the Genemorph II random mutagenesis kit (Stratagene). Four PCR reactions were used, with 1 ng pPM163 (containing scFv13 template) in each reaction. The resulting PCR product was digested with NdeI and BstBI, purified by gel electrophoresis, and cloned into the pSALectΔBla cassette digested with the same enzymes. The library was transformed into electrocompetent DH5α cells, and the library size and error rate were determined. The library size and error rate were determined to be 2.4x10⁶ members and ∼5 mutations per gene, respectively. The resulting plasmid library was then transformed into MC4100 cells for beadbased screening. After the first round of mutagenesis and screening, a second-generation library was constructed in the same manner using first-round clone 1-4 as a template. The library size and error rate for this library were determined to be 1.2x10⁶ members and ∼7 mutations per gene, respectively. Saturation mutagenesis of residue S55 in the VH domain of scFv13 was generated using the QuikChange II site-directed mutagenesis kit (Stratagene) with primers that included random bases at the VH S55 site to produce an NNK codon, where N is A, C, G, or T and K is G or T. After sequencing ∼60 randomly selected transformants, clones with all but three amino acids at position S55 were isolated. The remaining three amino acids (E, H, and K) were cloned individually using the same site-directed mutagenesis kit.

**Flow cytometry.** Flow cytometric analysis was used to detect membrane-anchored translocation intermediates and evaluate binding of anchored proteins to a target antigen, namely β-gal. β-Gal was labeled with FITC for these experiments using the FluoroTag FITC conjugate kit (Sigma). β-gal (Biochemika) was dissolved at 5 mg/mL in sodium bicarbonate buffer (0.1 M, pH 9.0), and then labeled following protocols provided with the FluoroTag conjugate kit. A molar extinction coefficient of 241590 M-1cm-1 was assumed for β-gal (Hoyoux A, et al. (2001) Appl Environ Microbiol 67(4):1529-1535). Following labeling, the molar ratio of fluorescent dye to β-gal (F/P ratio) was 2.3, and the final concentration of FITC-β-gal was 0.6 mg/mL. Spheroplasts were prepared as described above, and 50 µL of spheroplasts was mixed with 50 µL of PBS containing a FITC-conjugated anti-FLAG antibody (Sigma, 10 µg/mL final concentration) or with 50 µL of PBS supplemented with 2 µL of FITC-conjugated β-gal. Spheroplasts were incubated with the FITC conjugates for 45 min in the dark, washed with 400 µL of PBS, and resuspended in 500 µL of PBS. Flow cytometry was performed using a FACS Calibur flow cytometer (BD).

**Enzyme-linked immunosorbant assay.** To evaluate the binding of purified scFvs to β-gal by enzyme-linked immunosorbant assay (ELISA). ELISA plates were coated overnight at 4°C with 50 µL/well of β-gal in PBS (10 µg/mL). Plates were then blocked at room temperature for 2 h with 2% non-fat milk in PBS. After washing plates using PBS supplemented with 0.1% Tween 20 (PBST), purified protein samples diluted in PBS with 50 µg/mL BSA (PBS-BSA) were added to the plates (50 µL/well). Plates were incubated for 1 h at room temperature and then washed with PBST. Horseradish peroxidase (HRP)-conjugated anti-6x-His antibody (Abcam) in PBS-BSA was added to the plates (50 µL/well). After 1 h of incubation at room temperature, plates were washed and then incubated with SigmaFast OPD horseradish peroxidase substrate (HRP substrate; Sigma) for 20 min. The reaction was quenched with H2SO4, and the absorbance of the wells was measured. ELISAs with spheroplasts were performed in a similar manner; membrane-bound scFvs were detected with an HRP-conjugated anti-FLAG antibody (Abcam).

**Subcellular fractionation and Western blot analysis.** To prepare subcellular fractions for Western blot analysis, 3 mL of induced culture was pelleted and washed with subcellular fractionation buffer (SFB; 30 mM Tris-HCl, 1 mM EDTA, 0.6 M sucrose). Cells were resuspended in 1 mL SFB and then incubated for 20 min at room temperature. After adding 266 µL of 5 mM MgSO4, cells were incubated for 10 min on ice. Cells were spun down, and the supernatant was taken as the periplasmic fraction. The pellet was treated with BugBuster Master Mix protein extraction reagent (Novagen) for 5 min at room temperature. Following centrifugation at 16000 rcf at room temperature for 5 min, the second supernatant was taken as the cytoplasmic soluble fraction, and the pellet was the insoluble fraction. To prepare samples for analysis of cytoplasmic solubility, 5 mL of induced culture was pelleted and resuspended in 1 mL of BugBuster Master Mix protein extraction reagent. Samples were incubated for 20 min at room temperature and then spun down at 16000 rcf for 20 min at 4°C. The supernatant was taken as the soluble fraction. The pellet was washed with Tris-HCl (50 mM) with EDTA (1 mM) and resuspended in PBS with 2% SDS. After boiling for 10 min, the samples were centrifuged for 10 min at 16000 rcf. The supernatant was taken as the insoluble fraction. Proteins were separated by 12% SDS-polyacrylamide gels (Bio-Rad), and Western blotting was performed according to standard protocols. Briefly, proteins were transferred onto polyvinylidene fluoride (PVDF) membranes and membranes were probed with either anti-FLAG antibodies conjugated with HRP (Abcam) or anti-6x-His antibodies conjugated to HRP (Abcam).

**Protein purification.** After obtaining soluble fractions as described above, 6x-His-tagged scFvs were purified using protocols provided with Ni-NTA protein purification spin columns (Qiagen). After spin-column purification, eluted protein was further purified using a 100 kDa molecular weight cut-off column (Sartorius Stedim) to remove a high molecular weight impurity. Final purity of scFvs was confirmed by Coomassie staining of scFvs on an SDS-PAGE gel. Surface plasmon resonance (SPR). Purified scFvs were immobilized on a Biacore CM-5 sensor chip (GE Healthcare) for measuring binding to β-gal on a Biacore 2000 instrument. Running buffer was HBS-EP buffer (GE Healthcare), and the Biacore Amine Coupling Kit (GE Healthcare) was used to immobilize scFvs. Flow channels were activated with EDC and NHS. Purified scFv solutions (20 µg/mL in 10 mM sodium acetate, pH 4.0) were injected to immobilize the scFvs and any remaining reactive groups were then capped with ethanolamine. scFvs were immobilized to achieve a response of approximately 2000 RU following capping. Solutions of β-gal with concentrations ranging from 2 to 512 µg/mL were injected, and association and dissociation curves were collected. Data were analyzed using the BIAevaluation software.

### Results

**Anchoring Tat substrates to the IM.** A method was developed for anchoring Tat exported proteins to the periplasmic side of the IM *of E. coli.* Such a strategy would allow facile detection and functional interrogation of these proteins using a two-step strategy that involves permeabilizing *E. coli* cells followed by immunolabeling (Fig. 1a). Because Tat proteins are subject to folding quality control (DeLisa et al., supra; Fisher et al., supra), it was hypothesized that this procedure would have an in built fitness filter such that only correctly folded proteins would be displayed on the IM. To enable Tat-mediated membrane anchoring, a class of endogenous *E. coli* Tat substrates that possesses C-terminal transmembrane (-helices (TMs) and are thus C-tail anchored integral membrane proteins was first investigated (Hatzixanthis et al., (2003), supra). One example is HybO, a nonessential Tat substrate that assembles with HybC to form a hydrogenase respiratory complex. Previous studies demonstrated that a 22-residue TM at the extreme C-terminus of HybO was sufficient to anchor this subunit to the periplasmic side of the IM (Hatzixanthis et al., (2003), supra). Moreover, addition of the HybO C-tail to soluble proteins rendered these proteins membrane-bound. To determine if C-tail anchored HybO could be immunodectected on the periplasmic side of the IM, wildtype (wt) *E. coli* cells were induced to express HybO with an N-terminal FLAG tag (inserted just upstream of the C terminal TM) and then incubated with EDTA and lysozyme to disrupt the OM and cell wall. The resulting spheroplasts were mixed with a FITC-conjugated anti-FLAG antibody, and the cell fluorescence was determined by flow cytometry (FC). Spheroplasts expressing HybO-FLAG were highly fluorescent whereas those that had been treated with proteinase K (PK) prior to labeling or those expressing a version of HybO without a signal peptide were 60- and 32-times less fluorescent, respectively (Fig. 1b). The fluorescent signal from cells expressing HybO-FLAG was dependent on spheroplasting as labeling of untreated cells resulted in only background fluorescence. However, spheroplasts expressing a variant of HybO that lacked the C-tail anchor were highly fluorescent (Fig. 1b). Treatment with PK eliminated this fluorescence, as did expression of HybO without an export signal (Fig. 1b). The observation that HybO remained attached to the IM without a C-tail anchoring motif but not without a functional Tat export signal suggested that the N-terminal signal peptide served as a membrane anchor.

To determine whether this could be extended to other proteins, *E. coli* maltose binding protein (MBP) was anchored to the IM. MBP is a soluble protein that can be rerouted to the Tat pathway by replacing its native Sec-dependent signal with a Tat-dependent signal (e.g., ssTorA) (Blaudeck et al., (2003) J Bacteriol 185(9):2811-2819). Mature MBP was modified with an N-terminal ssTorA signal immediately followed by a FLAG epitope tag and a C-terminal HybO C-tail (HC) anchor motif. Spheroplasts expressing the ssTorA-FLAG-MBP-HC chimera were 14-and 9-times more fluorescent than unpermeabilized cells expressing the same construct or spheroplasts expressing the chimera without ssTorA, respectively (Fig. 5). PK treatment of spheroplasts was sufficient to eliminate the signal. Similar to HybO, immunodetection of MBP did not require the C-tail anchor (Fig. 5a). In fact, spheroplasts expressing ssTorA-FLAG-MBP were nearly twice as fluorescent as their ssTorA-FLAG-MBP-HC-expressing counterparts. Taken together, these data indicate that plasmid-expressed Tat substrates become anchored in the IM by their N-terminal signal peptide.

To determine if IM display of Tat substrates was regulated by the folding quality control feature of the bacterial Tat system (DeLisa et al., supra; Fisher et al., supra), the MBP domain in ssTorA-FLAG-MBP was modified with two amino acid substitutions, namely Gly32Asp and Ile33Pro. The resulting MBP variant, called MalE31, is highly aggregation prone and thus blocked for export via the Tat pathway (Fisher et al., supra). The fluorescence of spheroplasts expressing ssTorA-FLAG-MalE31 was indistinguishable from that of spheroplasts expressing ssTorA-FLAG-MBP (Fig. 5b), irrespective of whether a C-tail anchor was present or not. To determine if the position of the FLAG epitope was important, Constructs were generated in which the FLAG epitope was positioned C-terminally (ssTorA-MBP-FLAG).

When the FLAG epitope was repositioned at the C-terminus of MBP, only the correctly folded wt MBP could be immunodetected on spheroplasts (Fig. 5c). The inability to detect the C-terminal FLAG epitope on the misfolded MalE31 domain suggested that this domain was blocked for Tat export. Based on these results, it was contemplated that the labeling strategy was detecting two distinct translocation intermediates. These intermediates, called Ti-1 and Ti-2, were previously observed in the plant thylakoidal Tat system (Hou et al., supra) but have not been reported for the bacterial Tat system.

Formation of Ti-1 and Ti-2 requires development of membrane spanning segments, one of which is likely provided by the hydrophobic domain present in the Tat signal peptide (Hou et al., supra). Placement of the FLAG tag immediately after the signal peptide appears to position this epitope in a periplasmically-oriented hydrophilic region between the two presumed membrane segments (Fig. 2a). Since formation of Ti-1 is "unassisted" and does not depend on the Arg-Arg motif or functional Tat machinery (Hou et al., supra), this epitope is accessible regardless of whether the substrate protein is ultimately translocated to the periplasm. This explains why the export-competent wt MBP and the export-incompetent MalE31 could both be immunolabeled when the FLAG tag was positioned N-terminally. On the other hand, a C-terminal FLAG epitope is sequestered in the cytoplasm and only becomes accessible to immunolabeling if the protein is competent for Tat export. Indeed, when the FLAG tag was located at the C-terminus, only correctly folded wt MBP but not misfolded MalE31 was efficiently labeled. Interestingly, even though both Ti-1 and Ti-2 are naturally transient intermediates, HybO and MBP remained anchored to the IM for more than 24 h after spheroplasting. Thus, it is contemplated that that the high substrate expression levels used here saturated both the translocation machinery and signal peptidase I such that these intermediates were long-lived.

**Separate immunodetection of Ti-1 and Ti-2.** To further illustrate separate labeling of Ti-1 and Ti-2 and to eliminate any biases that may be introduced by using proteins such as HybO and MBP that are naturally exported in bacteria, a series of chimeras based on scFv13, a human antibody fragment specific for β-galactosidase (β-gal) were created (Martineau et al., (1998) J J Mol Biol 280(1):117-127).22). Previous studies demonstrated that wt scFv13, which folds poorly in the cytoplasm *of E. coli,* was incapable of Tat export (Fisher & DeLisa (2009) J Mol Biol 385(1):299-311). However, folding-enhanced variants of scFv13 (e.g., scFv13.R4) have been isolated (Martineau et al., supra), and these are efficiently localized to the periplasm by the Tat export machinery (Fisher and DeLisa, supra). In agreement with the model (Fig. 2a), both the poorly folded wt scFv13 and the folding enhanced scFv13.R4 could be detected on the IM when the FLAG tag was positioned between the signal peptide and the scFv domain (Fig. 2b). Consistent with the model for Ti-1 formation (Hou et al., supra, labeling proceeded even in the presence of a defective twin Lys signal peptide or in a host lacking the *tatC* gene (Fig. 2b), which encodes one of the essential components of the Tat translocase (Bogsch et al. supra). Only removal of the Tat signal peptide was sufficient to eliminate labeling (Fig. 2b). When the FLAG tag was moved to the C-terminus of each scFv, labeling of ssTorA9 scFv13.R4-FLAG resulted in a nearly 10-fold increase in fluorescence compared to the background level of fluorescence from the export-incompetent ssTorA-scFv13-FLAG (Fig. 2c). Ti-2 formation by scFv13.R4 was dependent on the Tat signal peptide (Fig. 2c). Unlike Ti-1, Ti-2 detection also depended on the conserved twin Arg residues in the signal peptide and the *tatC* gene (Fig. 2c). Thus, Ti-2 formation depended on both functional Tat targeting and on correct substrate folding, whereas Ti-1 formation was insensitive to these parameters.

**MAD-TRAP detection of ligand binding.** Collectively, the data above form the basis of a new technology called MAD-TRAP (membrane-anchored display for Tat-based recognition of associating proteins). To evaluate the utility of MAD-TRAP for detecting correctly folded *and* functional antibody fragments displayed on the IM, the ability of membrane tethered scFv13.R4 to bind β-gal was examined. To test this, spheroplasts expressing ssTorA-scFv13.R4-FLAG were mixed with β -gal that had been conjugated to FITC (β-gal-FITC) and analyzed by FC. Strong fluorescence was associated with spheroplasts (Fig. 3a), indicating binding of β -gal-FITC by the membrane-anchored antibody fragment. In contrast, no antigen binding was observed for wt scFv13 or for a version of scFv13.R4 that lacked a signal peptide, confirming that correct folding and Tat targeting are required for IM display. To confirm binding specificity, two unrelated scFv sequences were expressed: scFv-Dig that is specific for the cardiac glycoside digoxin and scFv-GCN4 that is specific for the bZIP domain of the yeast transcription factor Gcn4. The scFv-Dig antibody misfolds in the cytoplasm of wt *E*. *coli* (DeLisa et al., supra) and, as a result, was barely detected on the IM following immunolabeling with anti-FLAG-FITC antibodies (Fig. 3a). The misfolded scFv-Dig also did not bind to β -gal-FITC. In the case of scFv-GCN4, which was previously optimized for intracellular expression (der Maur AA, et al. (2002) J Biol Chem 277(47):45075-45085), expression on the IM was readily detected with anti-FLAG-FITC antibodies (Fig. 3a). However, correctly folded scFv-GCN4 that was displayed on the IM did not cross-react with β-gal-FITC, confirming the binding specificity of the assay. In parallel, it was observed that antigen-coated ELISA plates (or beads) could be used to readily discriminate correctly folded, functional antibody fragments from those that are incorrectly folded or improperly targeted to the Tat pathway (Fig. 3b). Western blot analysis of subcellular fractions confirmed that only the folding-enhanced scFv13.R4 was exported from the cytoplasm and that export depended on a functional signal peptide (Fig. 3c).

**Combinatorial library screening using MAD-TRAP.** Based on the above findings, the use of MAD-TRAP for screening combinatorial libraries of antibody fragments was investigated. Enrichment experiments where mixtures of treated cells were labeled with β-gal-FITC followed by a single round of biopanning using β-gal coated magnetic beads were first performed. When spheroplasts expressing ssTorA-scFv13.R4-FLAG were mixed 1:1 or 1:100 with spheroplasts cells expressing ssTorA-MBP-FLAG and panned on β-gal, 100% of the recovered clones were identified as ssTorA-scFv13.R4-FLAG (Fig. 6a). Enrichment was similarly achieved when ssTorA-scFv13.R4-FLAG was mixed 1:1 or 1:100 with ssTorA-scFv- GCN4-FLAG (Fig. 6b).

Next, a directed evolution strategy was used to engineer variants of wt scFv13 that exhibited improved expression and/or antigen binding. For this, the gene encoding wt scFv13 was mutagenized by error-prone PCR (Fromant et al., (1995) Anal Biochem 224(1):347-353), and the PCR products were cloned between the ssTorA sequence and a FLAG epitope tag. The resulting plasmid DNA library was then transformed into *E. coli,* giving rise to 2.4 106 independent clones. DNA sequencing of 12 library clones selected at random revealed an average of ∼0.5% nucleotide substitutions per gene. Cells expressing the ssTorA-scFv13-FLAG library were treated with EDTA-lysozyme and mixed with β-gal-coated magnetic beads. Bead-bound spheroplasts were isolated, and the genes encoding the antigen-binding scFvs were rescued by PCR amplification of the DNA from the isolated cells. This was aided by the fact that the conditions used for PCR amplification result in the quantitative release of cellular DNA from the cells that have partially hydrolyzed cell walls due to the EDTA-lysozyme treatment during labeling (Harvey BR, et al. (2004) Proc Natl Acad Sci U S A 101(25):9193-9198). Direct PCR amplification of scFv sequences rather than cell plating was used to recover positive hits, because the plating efficiency of isolated clones was low, as reported previously for EDTA7 lysozyme treated *E. coli* cell libraries (Harvey et al., supra). After 30 rounds of PCR amplification, the isolated scFv sequences were cloned back into the original plasmid backbone and transformed into fresh *E. coli.* The resulting sublibrary was subjected to an additional round of biopanning exactly as above. FC screening of the library cells prior to enrichment as well as cells isolated from the first and second rounds of biopanning revealed a clear enrichment in both cell surface expression and antigen binding (Fig. 6c). Next, 30 clones from the second round were randomly chosen for rescreening using FC. Following β -gal-FITC labeling, fluorescence for 29 of these clones was confirmed to be significantly greater than the parental wt scFv13 clone. The most active of these, clone 1-4, was chosen for further characterization. Sequencing of this clone revealed only a single S55R substitution in complementarity determining region 2 (CDR2) of the heavy chain (V_{H}; Table 1 and Fig. 7), which is also one of the 7 mutations isolated previously in scFv13.R4 (Martineau et al., (1998), supra). To determine the effect of this substitution on *in vivo* folding and activity, the cytoplasmic expression of clone 1-4 versus its progenitor wt scFv13 was compared. Western blot analysis revealed that clone 1-4 was much more soluble in the cytoplasm than wt scFv13 when each was expressed from plasmid pET-21a(+) without an N-terminal Tat signal peptide (Fig. 4a). The binding activity and affinity of 1-4 and wt scFv13 following their purification from the cytoplasm was next compared. By ELISA, clone 1-4 exhibited a significant increase in binding activity compared to wt scFv13 (Fig. 4b). Likewise, surface plasmon resonance revealed a 50% increase in affinity for clone 1-4 compared to wt scFv13 (Table 1). These results indicate that just a single amino acid substitution in clone 1-4 was sufficient to enhance both *in vivo* folding and affinity for its cognate antigen and indicate an unexpectedly short evolutionary distance between a stable, well folded scFv and its less stable, poorly expressed parental sequence. The importance of this residue was highlighted by the fact that substitution of most amino acids in position S55 resulted in complete loss of binding activity (Fig. 8), which in some but not all cases was due to poor solubility. However, an S55K mutant bound to β-gal at a level that rivaled the S55R mutant, indicating that the improvement in binding and solubility of clone 1-4 may be due to the introduction of a positive charge in this position.

**Affinity maturation of clone 1-4 using MAD-TRAP.** To determine whether MAD-TRAP could be used to further improve the expression and/or binding activity of clone 1-4, an additional round of mutagenesis and screening was performed. An error-prone library of clone 1-4 was created as described above. However, to favor the isolation of higher affinity clones, competitive biopanning in the presence of purified 1-4 protein that served as a competitor for β - gal binding was performed. Following this procedure, 13 candidates were identified of which 10 were determined to be true positives by ELISA-based rescreening. The two most active clones from this group, namely clones 2-1 and 2-3, were characterized in more detail. A total of 3 and 6 additional mutations were acquired by clones 2-1 and 2-3, respectively (Table 1). The mutations in clone 2-1 were clustered in the light chain (V_{L}) only and included the G51D mutation in CDR2 that is also present in scFv13.R4. Clone 2-3 also carried a V_{L} G51D substitution as well as mutations in the V_{L} CDRs and the V_{H} frameworks. The V_{H} framework mutations were identical to (e.g., V48I), similar to (e.g., A93T) or nearby to (e.g., L11P) substitutions in scFv13-R4. The effect of these mutations on folding and activity is clearly distinct. For instance, clone 2-1 showed no detectable increase in cytoplasmic expression and a small decrease in the amount of protein that partitioned to the insoluble fraction compared to its parent 1-4 (Fig. 4a). However, binding activity and affinity of this clone increased to a level that rivaled scFv13.R4 (Fig. 4b and Table S1). In the case of clone 2-3, the binding properties showed a more modest increase compared to parental clone 1-4 (Fig. 4b); *but in vivo* folding was dramatically improved compared to clone 1-4 as evidenced by a large increase in soluble expression and no detectable accumulation in the insoluble fraction (Fig. 4a). Collectively, these results show the Tat mechanism can be leveraged for the selection of binding proteins with significant improvements in both *in vivo* folding efficiency and antigen binding activity.

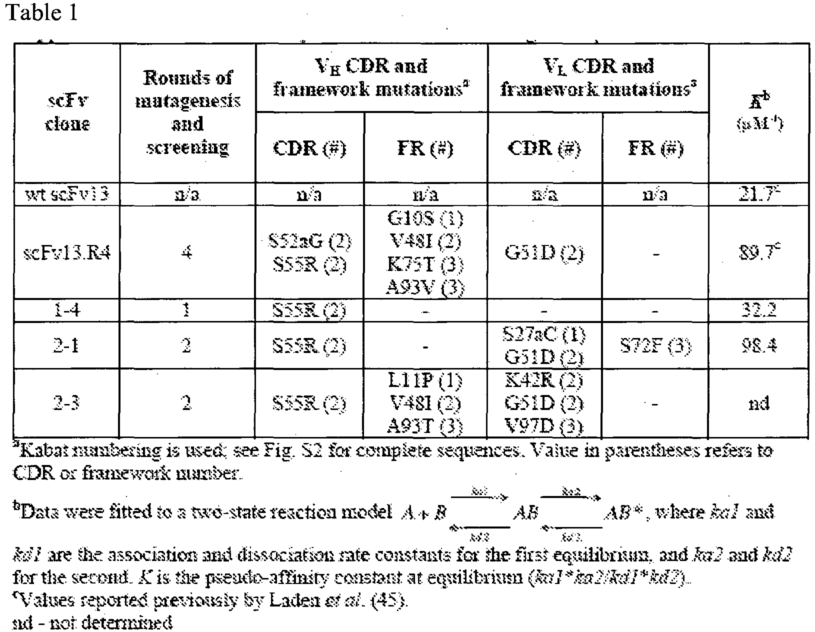

Two long-lived Tat translocation intermediates, Ti-1 and Ti-2, that can be detected on the IM of permeabilized *E. coli* cells and are likely to be equivalent to Ti-1 and Ti-2 previously identified for the plant thylakoidal Tat system were identified (Berghofer and Klosgen, supra; Hou et al., supra). These results help dissect the transport process into several distinct steps that are characterized by separate translocation intermediates. For instance, detection of Ti-1 indicates that in the case of the Tat substrates tested here, the precursor assumes a loop-like structure involving the signal peptide and the early part of the mature region, leaving the N- and C-termini at the cytoplasmic face. Such an insertion mechanism is not unique to the Tat pathway as certain Sec pathway substrates such as OmpA have long been known to transiently adopt loop-like conformations (Kuhn A, et al. (1994) Eur JBiochem 226(3):891-897). Ti-1 formation in *E. coli* can proceed with a nonfunctional Lys-Lys signal peptide or in the absence of proteinaceous transport machinery, similar to the situation in plant thylakoids. This indicates that initial membrane insertion and adoption of the transient loop topology for at least some *E. coli* Tat substrates occurs spontaneously. A similar spontaneous membrane insertion mechanism has been described for several thylakoidal membrane proteins that do not rely on the Tat (or Sec) protein export systems (Kim et al., (1999) J Biol Chem 274(8):4715-4721; Michl et al., (1994) EMBO J 13(6):1310-1317). Following formation of Ti-1, proteins that are competent for export (e.g., have a functional Arg-Arg signal peptide and are correctly/completely folded) undergo transition to a bitopic topology during which the C-terminus is translocated across the membrane while the N8 terminus remains in the cytoplasm. The fact that both folded and misfolded substrates formed the Ti-1 conformation, whereas only folded substrates formed Ti-2 indicates that any interrogation of substrate folding state likely occurs after insertion into the inner membrane, either preceding or coincident with the Ti-1 to Ti-2 transition. Moreover, since Ti-2 formation (but not Ti-1) is dependent on *tatC,* the Tat translocase may contribute to the quality control mechanism. In line with this hypothesis, the TatBC proteins were previously shown to interact with both folded and unfolded Tat substrates (Panahandeh et al., (2008) J Biol Chem 283(48):33267-33275.). However, site-specific cross-linking revealed a perturbed interaction between the signal peptide of the unfolded precursor and the TatBC receptor site, consistent with some degree of quality control by TatBC. Since TatB molecules oligomerize to form a transient cytoplasmic binding site for folded Tat substrates (Maurer et al., Mol Biol Cell 21(23):4151-4161), Ti-1 intermediates could access the TatB binding site prior to membrane translocation and formation of Ti-2.

The Ti-2 intermediate was used to create MAD-TRAP - a new protein engineering platform that enables simultaneous engineering of the solubility and antigen binding properties of an scFv antibody. Because a Tat substrate must pass an in-built fitness filter to form Ti-2 and become displayed on the inner membrane, the MAD-TRAP technique described herein effectively eliminates poorly folded scFv clones prior to panning for antigen-binding. This is useful for the development of scFv antibodies that can be expressed in an intracellular compartment (e.g., intrabodies). Intrabodies have shown great potential as therapeutics for infectious diseases, neurodegenerative disorders and cancer (Williams BR & Zhu Z (2006) Curr Med Chem 13(12):1473-1480; Miller TW & Messer A (2005) Mol Ther 12(3):394-401; Marasco et al., (1999) J Immunol Methods 231(1-2):223-238); they are even being tested in cancer clinical trials (Alvarez RD, et al. (2000) Clin Cancer Res 6(8):3081-3087). However, generation of intrabodies is challenging because formation of the disulfide bonds connecting the two β-sheets in each of the V_{H} and V_{L} domains is disfavored in the reducing environment of the cytoplasm. The absence of these bonds causes a large decrease in the ΔG of folding (∼4-5 kcal/mol) (Frisch et al. (1996) Fold Des 1(6):431-440) and accompanied loss of antigen binding activity, susceptibility to proteolysis, and aggregation (Cattaneo A & Biocca S (1999) Trends Biotechnol 17(3):115-121; Proba et al., (1997) J Mol Biol 265(2):161-172). The MAD-TRAP strategy simplifies the generation of scFv variants that are well suited to function as intrabodies. Unlike most existing platforms, the incorporation of the Tat folding quality control allows screening for antigen binding and intracellular solubility in a single step.

This point is best illustrated by clone 1-4, which was isolated after just a single round of mutagenesis and screening and exhibited marked improvements in both soluble expression and binding affinity. MAD-TRAP exploits the Tat folding quality control mechanism to screen antibody libraries but in a notable departure does not require intracellular expression of the antigen. Hence, MAD-TRAP represents a useful new tool for the antibody engineering toolbox that permits isolation of intrabodies against more challenging targets such as post-translationally modified proteins (e.g., phosphoproteins) or integral membrane proteins for which *in vitro* panning is possible.

## Claims

1. A method of screening mutants of a target protein for a desired property comprising:
displaying a library comprising a plurality of host cells, wherein each host cell expresses a heterologous fusion protein on the inner membrane of host cells, wherein said heterologous fusion protein comprises segments encoding a Tat signal operably linked to a mutagenized target protein, wherein said Tat signal sequence is on the N-terminal of the fusion protein;
forming spheroplasts from said host cells;
contacting said spheroplasts with a target protein binding partner; and
selecting spheroplasts that bind said target protein binding partner.

2. The method of Claim 1, wherein said mutagenized target proteins are selected from the group consisting of antigen binding proteins, receptor proteins and receptor ligand proteins.

3. The method of Claim 2, wherein said antigen binding molecule is an scFv or an intrabody.

4. The method of Claim 1, wherein said target protein binding partner is selected from the group consisting of a molecule comprising an epitope, a receptor protein, and a receptor ligand.

5. The method of Claim 4, wherein said target protein binding partner is displayed on a solid support.

6. The method of Claim 1, wherein said mutagenized target proteins are encoded by nucleic acids mutagenized by an amplification based mutagenesis procedure.

7. The method of Claim 1, further comprising the step of isolating DNA encoding said mutagenized target protein from said spheroplasts that bind said target protein binding partner.

8. The method of Claim 7, further comprising:
subjecting DNA encoding said mutagenized target protein to a second round of mutagenesis to provide a second mutagenized target protein nucleic acid library,
expressing said second mutagenized target protein nucleic acid library in a host cell,
forming spheroplasts from said host cells;
contacting said spheroplasts with a target protein binding partner; and
selecting spheroplasts that bind said target protein binding partner.

9. The method of Claim 8, further comprising the step of isolating DNA encoding said mutagenized target protein from said spheroplasts that bind said target protein binding partner.

10. The method of Claim 7, wherein said mutagenized protein exhibits a property selected from the group consisting of enhanced solubility, intracellular folding efficiency, binding affinity for said target protein binding partner, and combinations thereof.

11. The method of Claim 1, wherein said contacting said spheroplasts with target protein binding partner includes contacting with a competitive binding partner.

12. The method of Claim 1, wherein said fusion protein comprises a protein tag at the C-terminus of said fusion protein.

13. The method of Claim 12, further comprising contacting said spheroplasts with a reagent specific for said protein tag wherein said selecting further comprises selecting spheroplasts that bind both said binding partner and said reagent specific for said protein tag.

14. A method of screening mutants of a target protein for a desired property comprising:
a) expressing in host cells a library of target nucleic acid molecules encoding fusion proteins comprising a Tat signal sequence operably linked to a mutated target protein so that said fusion proteins are displayed on the inner membrane of said host cells,
b) forming spheroplasts from said host cells;
c) contacting said spheroplasts with a target protein binding partner;
d) selecting spheroplasts that bind said target protein binding partner; and
e) isolating a target nucleic acid molecule encoding said mutated target protein from said spheroplasts that bind said target protein binding partner, wherein said mutagenized protein exhibits a property selected from the group consisting of enhanced solubility, intracellular folding efficiency, binding affinity for said target protein binding partner, and combinations thereof.

15. The method of Claim 14, further comprising
f) mutagenizing said nucleic acid molecule isolated in step e of Claim 14 and operably linking said nucleic acid molecules to a Tat signal sequence to provide a second library of mutagenized target nucleic acid molecules, and
g) repeating steps b-e of Claim 14 and, optionally:
h) mutagenizing said nucleic acid molecule isolated in step g of Claim 15 and operably linking said nucleic acid molecules to a Tat signal sequence to provide a third library of mutagenized target nucleic acid molecules, and
i) repeating steps b-e of Claim 14.

16. The method of Claim 14, wherein said mutagenized target proteins are selected from the group consisting of antigen binding proteins, receptor proteins and receptor ligand proteins.

17. The method of Claim 16, wherein said antigen binding molecule is an scFv or an intrabody.

18. The method of Claim 14, wherein said target protein binding partner is selected from the group consisting of a molecule comprising an epitope, a receptor protein, and a receptor ligand and wherein said target protein binding partner is displayed on a solid support.

19. The method of claim 14 comprising:
a) expressing in host cells a library of target nucleic acid molecules encoding fusion proteins comprising a Tat signal sequence and a protein tag operably linked to a mutated target protein so that said fusion proteins are displayed on the inner membrane of said host cells,
b) forming spheroplasts from said host cells;
c) contacting said spheroplasts with a target protein binding partner and reagent specific for said protein tag;
d) selecting spheroplasts that bind said target protein binding partner and said reagent specific for said protein tag; and
e) isolating a target nucleic acid molecule encoding said mutated target protein from said spheroplasts that bind said target protein binding partner, wherein said mutagenized protein exhibits a property selected from the group consisting of enhanced solubility, intracellular folding efficiency, binding affinity for said target protein binding partner, and combinations thereof.

20. A library of spheroplasts comprising a library of target nucleic acid molecules encoding heterologous fusion proteins comprising a Tat signal sequence operably linked to a mutated target protein so that said fusion proteins are displayed on the inner membrane of said spheroplasts.

## Patentansprüche

1. Verfahren zum Screening von Mutanten eines Zielproteins auf ein gewünschtes Merkmal, umfassend:
Auflegen einer eine Vielzahl an Wirtszellen umfassenden Bibliothek, wobei jede Wirtszelle an der Innenmembran der Wirtszellen ein heterologes Fusionsprotein exprimiert, wobei besagtes heterologes Fusionsprotein Segmente umfasst, die ein Tat-Signal kodieren, das operabel mit einem mutagenisierten Zielprotein verbunden ist, wobei besagte Tat-Signalsequenz am N-terminalem Ende des Fusionsproteins ist;
Bildung von Sphäroplasten aus besagten Wirtszellen;
Kontaktieren besagter Sphäroplasten mit einem Zielproteinbindungspartner; und
Selektion der Sphäroplasten, die besagten Zielproteinbin-dungspartner binden.

2. Verfahren nach Anspruch 1, wobei besagte mutagenisierte Zielproteine ausgewählt sind aus der Gruppe bestehend aus Anti-genbindungsproteinen, Rezeptorproteinen und Rezeptorliganden-proteinen.

3. Verfahren nach Anspruch 2, wobei besagtes Antigenbindungsmolekül ein scFv oder ein Intrabody ist.

4. Verfahren nach Anspruch 1, wobei besagter Zielproteinbin-dungspartner ausgewählt ist aus der Gruppe bestehend aus einem ein Epitop umfassenden Molekül, einem Rezeptorprotein und einem Rezeptorliganden.

5. Verfahren nach Anspruch 4, wobei besagter Zielproteinbin-dungspartner auf einer festen Unterlage vorliegt.

6. Verfahren nach Anspruch 1, wobei besagte mutagenisierte Zielproteine durch Nukleinsäuren kodiert werden, die durch ein Amplifikation-basiertes Mutageneseverfahren mutagenisiert wur-den.

7. Verfahren nach Anspruch 1, des Weiteren umfassend den Schritt der Isolierung der DNS, die besagtes mutagenisiertes Zielprotein kodiert, aus den Sphäroplasten, die besagten Ziel-proteinbindungspartner binden.

8. Verfahren nach Anspruch 7, des Weiteren umfassend:
Aussetzung der besagtes Zielprotein kodierenden DNS einer zweiten Mutageneserunde um eine zweite mutagenisierte Zielpro-teinnukleinsäurebibliothek bereit zu stellen,
Expression besagter zweiter mutagenisierter Zielprotein-nukleinsäurebibliothek in einer Wirtszelle,
Bildung von Sphäroplasten aus besagten Wirtszellen;
Kontaktieren besagter Sphäroplasten mit einem Zielproteinbindungspartner; und
Selektion der Sphäroplasten, die besagten Zielproteinbin-dungspartner binden.

9. Verfahren nach Anspruch 8, des Weiteren umfassend den Schritt der Isolierung der DNS, die besagtes mutagenisiertes Zielprotein kodiert, aus den Sphäroplasten, die besagten Ziel-proteinbindungspartner binden.

10. Verfahren nach Anspruch 7, wobei besagtes mutagenisiertes Protein ein Merkmal zeigt, das ausgewählt ist aus der Gruppe bestehend aus verstärkter Löslichkeit, intrazellulärer Fal-tungseffizienz, Bindungsaffinität in Bezug auf besagten Ziel-proteinbindungspartner und Kombinationen davon.

11. Verfahren nach Anspruch 1, wobei besagtes Kontaktieren besagter Sphäroplasten mit dem Zielproteinbindungspartner das Kontaktieren mit einem konkurrierenden Bindungspartner ein-schließt.

12. Verfahren nach Anspruch 1, wobei besagtes Fusionsprotein ein Proteintag am C-terminalen Ende des besagten Fusionspro-teins umfasst.

13. Verfahren nach Anspruch 12, des Weiteren umfassend das Kontaktieren besagter Sphäroplasten mit einem Reagenz, das für besagtes Proteintag spezifisch ist, wobei besagte Selektion des Weiteren die Selektion der Sphäroplasten umfasst, die beides binden, den besagten Bindungspartner und besagtes Reagenz, das für besagtes Proteintag spezifisch ist.

14. Verfahren zum Screening von Mutanten eines Zielproteins auf ein gewünschtes Merkmal, umfassend:
a) Expression in einer Wirtszelle einer Bibliothek von Zielnukleinsäuremolekülen, die Fusionsproteine kodieren, die eine Tat-Signalsequenz umfassen, die operabel mit einem mutierten Zielprotein verbunden ist, so dass besagte Fusionsproteine an der Innenmembran besagter Wirtszellen vorliegen,
b) Bildung von Sphäroplasten aus besagten Wirtszellen;
c) Kontaktieren besagter Sphäroplasten mit einem Zielpro-teinbindungspartner;
d) Selektion der Sphäroplasten, die besagten Zielprotein-bindungspartner binden; und
e) Isolierung eines Zielnukleinsäuremoleküls, das besag-tes mutiertes Zielprotein kodiert, aus den Sphäroplasten, die besagten Zielproteinbindungspartner binden, wobei besagtes mutagenisiertes Protein ein Merkmal zeigt, das ausgewählt ist aus der Gruppe bestehend aus verstärkter Löslichkeit, intrazellulä-rer Fal-tungseffizienz, Bindungsaffinität in Bezug auf besagten Zielproteinbindungspartner und Kombinationen davon.

15. Verfahren nach Anspruch 14, des Weiteren umfassend
f) Mutagenese des besagten, in Schritt e von Anspruch 14 isolierten, Nukleinsäuremoleküls und operable Verbindung besag-ter Nukleinsäuremoleküle mit einer Tat-Signalsequenz um eine zweite Bibliothek von mutagenisierten Zielnukleinsäuremolekülen bereit zu stellen, und
g) Wiederholung der Schritte b-e von Anspruch 14 und, optional:
h) Mutagenese von besagten, in Schritt g von Anspruch 15 isolierten, Nukleinsäuremoleküls und operable Verbindung besag-ter Nukleinsäuremoleküle mit einer Tat-Signalsequenz um eine dritte Bibliothek an mutagenisierten Zielnukleinsäuremolekülen bereit zu stellen, und
i) Wiederholung der Schritte b-e von Anspruch 14.

16. Verfahren nach Anspruch 14, wobei besagte mutagenisierte Zielproteine ausgewählt sind aus der Gruppe bestehend aus Anti-genbindungsproteinen, Rezeptorproteinen und Rezeptorliganden-proteinen.

17. Verfahren nach Anspruch 16, wobei besagtes Antigenbindungsmolekül ein scFv oder ein Intrabody ist.

18. Verfahren nach Anspruch 14, wobei besagter Zielprotein-bindungspartner ausgewählt ist aus der Gruppe bestehend aus einem ein Epitop umfassenden Molekül, einem Rezeptorprotein und einem Rezeptorliganden und wobei besagter Zielproteinbindungs-partner auf einer festen Unterlage vorliegt.

19. Verfahren nach Anspruch 14, umfassend:
a) Expression in Wirtszellen einer Bibliothek von Ziel-nukleinsäuremolekülen, die Fusionsproteine kodieren, die eine Tat-Signalsequenz und ein Proteintag umfassen, das mit einem mutierten Zielprotein operabel verbunden ist, so dass besagte Fusionsproteine an der Innenmembran besagter Wirtszellen vor-liegen,
b) Bildung von Sphäroplasten aus besagten Wirtszellen;
c) Kontaktieren besagter Sphäroplasten mit einem Zielpro-teinbindungspartner und einem Reagenz, das für besagtes Pro-teintag spezifisch ist;
d) Selektion der Sphäroplasten, die besagten Zielprotein-bindungspartner und besagtes Reagenz, das für besagtes Protein-tag spezifisch ist, binden; und
e) Isolierung eines Zielnukleinsäuremoleküls, das besag-tes mutiertes Zielprotein kodiert, aus den Sphäroplasten, die besagten Zielproteinbindungspartner binden, wobei besagtes mutagenisiertes Protein ein Merkmal zeigt, das ausgewählt ist aus der Gruppe bestehend aus verstärkter Löslichkeit, intrazellulä-rer Fal-tungseffizienz, Bindungsaffinität in Bezug auf besagten Zielproteinbindungspartner und Kombinationen davon.

20. Bibliothek von Sphäroplasten umfassend eine Bibliothek von Zielnukleinsäuremolekülen, die heterologe Fusionsproteine kodieren, die eine Tat-Signalsequenz umfassen, die operabel mit einem mutierten Zielprotein verbunden ist, so dass besagte Fu-sionsproteine an der Innenmembran besagter Sphäroplasten vor-liegen.

## Revendications

1. Procédé de criblage de mutants d'une protéine cible pour identifier une propriété souhaitée, comprenant :
la présentation d'une banque comprenant une pluralité de cellules hôtes, où chaque cellule hôte exprime une protéine de fusion hétérologue sur la membrane interne des cellules hôtes, où ladite protéine de fusion hétérologue comprend des segments codant pour un signal Tat en liaison fonctionnelle avec une protéine cible ayant subi une mutagenèse, où ladite séquence signal Tat est sur l'extrémité N-terminale de la protéine de fusion ;
la formation de sphéroplastes à partir desdites cellules hôtes ;
la mise en contact desdits sphéroplastes avec un partenaire de liaison de la protéine cible ; et
la sélection de sphéroplastes qui se lient audit partenaire de liaison de la protéine cible.

2. Procédé selon la revendication 1, dans lequel lesdites protéines cibles ayant subi une mutagenèse sont sélectionnées dans le groupe consistant en des protéines de liaison à un antigène, des protéines de récepteur et des protéines de ligand d'un récepteur.

3. Procédé selon la revendication 2, dans lequel ladite molécule de liaison à un antigène est un scFv ou un anticorps intracellulaire (intracorps).

4. Procédé selon la revendication 1, dans lequel ledit partenaire de liaison de la protéine cible est sélectionné dans le groupe consistant en une molécule comprenant un épitope, une protéine de récepteur, et un ligand d'un récepteur.

5. Procédé selon la revendication 4, dans lequel ledit partenaire de liaison de la protéine cible est présenté sur un support solide.

6. Procédé selon la revendication 1, dans lequel lesdites protéines cibles ayant subi une mutagenèse sont codées par des acides nucléiques ayant subi une mutagenèse par une procédure de mutagenèse basée sur une amplification.

7. Procédé selon la revendication 1, comprenant en outre l'étape d'isolement d'un ADN codant pour ladite protéine cible ayant subi une mutagenèse à partir desdits sphéroplastes qui se lient audit partenaire de liaison de la protéine cible.

8. Procédé selon la revendication 7, comprenant en outre:
le fait de soumettre un ADN codant pour ladite protéine cible ayant subi une mutagenèse à un second tour de mutagenèse afin d'obtenir une deuxième banque d'acides nucléiques d'une protéine cible ayant subi une mutagenèse,
l'expression de ladite deuxième banque d'acides nucléiques d'une protéine cible ayant subi une mutagenèse dans une cellule hôte,
la formation de sphéroplastes à partir desdites cellules hôtes ;
la mise en contact desdits sphéroplastes avec un partenaire de liaison de la protéine cible ; et
la sélection de sphéroplastes qui se lient audit partenaire de liaison de la protéine cible.

9. Procédé selon la revendication 8, comprenant en outre l'étape d'isolement d'un ADN codant pour ladite protéine cible ayant subi une mutagenèse à partir desdits sphéroplastes qui se lient audit partenaire de liaison de la protéine cible.

10. Procédé selon la revendication 7, dans lequel ladite protéine ayant subi une mutagenèse exhibe une propriété sélectionnée dans le groupe consistant en une solubilité améliorée, une efficacité de repliement intracellulaire, une affinité de liaison pour ledit partenaire de liaison de la protéine cible, et des combinaisons de celles-ci.

11. Procédé selon la revendication 1, dans lequel ladite mise en contact desdits sphéroplastes avec un partenaire de liaison de la protéine cible comprend une mise en contact avec un partenaire de liaison compétitif.

12. Procédé selon la revendication 1, dans lequel ladite protéine de fusion comprend un marqueur protéique à l'extrémité C-terminale de ladite protéine de fusion.

13. Procédé selon la revendication 12, comprenant en outre la mise en contact desdits sphéroplastes avec un réactif spécifique pour ledit marqueur protéique, où ladite sélection comprend en outre la sélection de sphéroplastes qui se lient à la fois audit partenaire de liaison et audit réactif spécifique pour ledit marqueur protéique.

14. Procédé de criblage de mutants d'une protéine cible pour identifier une propriété souhaitée, comprenant :
a) l'expression, dans des cellules hôtes, d'une banque de molécules d'acide nucléique cibles codant pour des protéines de fusion comprenant une séquence signal Tat en liaison fonctionnelle avec une protéine cible mutée de sorte que lesdites protéines de fusion soient présentées sur la membrane interne desdites cellules hôtes,
b) la formation de sphéroplastes à partir desdites cellules hôtes ;
c) la mise en contact desdits sphéroplastes avec un partenaire de liaison de la protéine cible ;
d) la sélection de sphéroplastes qui se lient audit partenaire de liaison de la protéine cible ; et
e) l'isolement d'une molécule d'acide nucléique cible codant pour ladite protéine cible mutée à partir desdits sphéroplastes qui se lient audit partenaire de liaison de la protéine cible, où ladite protéine ayant subi une mutagenèse exhibe une propriété sélectionnée dans le groupe consistant en une solubilité améliorée, une efficacité de repliement intracellulaire, une affinité de liaison pour ledit partenaire de liaison de la protéine cible, et des combinaisons de celles-ci.

15. Procédé selon la revendication 14, comprenant en outre
f) le fait de réaliser une mutagenèse de ladite molécule d'acide nucléique isolée à l'étape e de la revendication 14 et de lier de manière fonctionnelle lesdites molécules d'acide nucléique à une séquence signal Tat afin d'obtenir une deuxième banque de molécules d'acide nucléique cibles ayant subi une mutagenèse, et
g) la répétition des étapes b-e de la revendication 14
et, facultativement :
h) le fait de réaliser une mutagenèse de ladite molécule d'acide nucléique isolée à l'étape g de la revendication 15 et de lier de manière fonctionnelle lesdites molécules d'acide nucléique à une séquence signal Tat afin d'obtenir une troisième banque de molécules d'acide nucléique cibles ayant subi une mutagenèse, et
i) la répétition des étapes b-e de la revendication 14.

16. Procédé selon la revendication 14, dans lequel lesdites protéines cibles ayant subi une mutagenèse sont sélectionnées dans le groupe consistant en des protéines de liaison à un antigène, des protéines de récepteur et des protéines de ligand d'un récepteur.

17. Procédé selon la revendication 16, dans lequel ladite molécule de liaison à un antigène est un scFv ou un anticorps intracellulaire (intracorps).

18. Procédé selon la revendication 14, dans lequel ledit partenaire de liaison de la protéine cible est sélectionné dans le groupe consistant en une molécule comprenant un épitope, une protéine de récepteur, et un ligand d'un récepteur, et dans lequel ledit partenaire de liaison de la protéine cible est présenté sur un support solide.

19. Procédé selon la revendication 14, comprenant :
a) l'expression, dans des cellules hôtes, d'une banque de molécules d'acide nucléique cibles codant pour des protéines de fusion comprenant une séquence signal Tat et un marqueur protéique en liaison fonctionnelle avec une protéine cible mutée de sorte que lesdites protéines de fusion soient présentées sur la membrane interne desdites cellules hôtes,
b) la formation de sphéroplastes à partir desdites cellules hôtes ;
c) la mise en contact desdits sphéroplastes avec un partenaire de liaison de la protéine cible et un réactif spécifique pour ledit marqueur protéique ;
d) la sélection de sphéroplastes qui se lient audit partenaire de liaison de la protéine cible et audit réactif spécifique pour ledit marqueur protéique ; et
e) l'isolement d'une molécule d'acide nucléique cible codant pour ladite protéine cible mutée à partir desdits sphéroplastes qui se lient audit partenaire de liaison de la protéine cible, où ladite protéine ayant subi une mutagenèse exhibe une propriété sélectionnée dans le groupe consistant en une solubilité améliorée, une efficacité de repliement intracellulaire, une affinité de liaison pour ledit partenaire de liaison de la protéine cible, et des combinaisons de celles-ci.

20. Banques de sphéroplastes comprenant une banque de molécules d'acide nucléique cibles codant pour des protéines de fusion hétérologues comprenant une séquence signal Tat en liaison fonctionnelle avec une protéine cible mutée de sorte que lesdites protéines de fusion soient présentées sur la membrane interne desdits sphéroplastes.
